(19) Europäisches Patentamt
European Patent Office
Office européen des brevets

(11) Veröffentlichungsnummer: **0 431 480 A2**

(12) # EUROPÄISCHE PATENTANMELDUNG

(21) Anmeldenummer: **90122907.0**

(22) Anmeldetag: **30.11.90**

(51) Int. Cl.5: **C07D 493/04**, A61K 31/335, C07F 7/18, C07H 17/08, //(C07D493/04,313:00,303:00)

(30) Priorität: **05.12.89 DE 3940131**

(43) Veröffentlichungstag der Anmeldung: **12.06.91 Patentblatt 91/24**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Granzer, Ernold, Dr.**
**Falkensteiner Strasse 24**
**W-6233 Kelkheim/Taunus(DE)**
Erfinder: **Hammann, Peter, Dr.**
**Bürgermeister-Rühl-Strasse 20**
**W-6113 Babenhausen(DE)**
Erfinder: **Kirsch, Reinhard, Dr.**
**Johannesallee 18**
**W-6230 Frankurt am Main(DE)**

(54) **Substituierte 10-Ring-Lactone, Verfahren zu deren Herstellung und Verwendung derselben.**

(57) Neue 10-Ring-Lactonderivate der Formeln I und II,

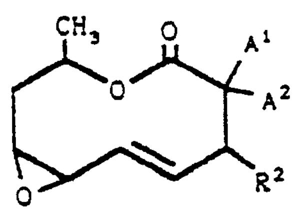
(I)

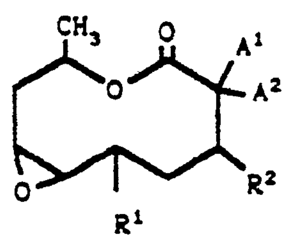
(II)

Verfahren zur Herstellung dieser Verbindungen, pharmazeutische Mittel, welche die erfindungsgemäßen aktiven Verbindungen enthalten und deren Verwendung als Arzneimittel mit insbesondere lipidsenkender Wirkung werden beschrieben.

EP 0 431 480 A2

## SUBSTITUIERTE 10-RING-LACTONE, VERFAHREN ZU DEREN HERSTELLUNG UND VERWENDUNG DERSELBEN

Die Erfindung betrifft substituierte 10-Ring Lactone, Verfahren zu ihrer Herstellung sowie deren Verwendung, insbesondere als Arzneimittel, welche die Cholesterin-Biosynthese inhibieren. Die erfindungsgemäßen Verbindungen können dementsprechend als Lipidsenker eingesetzt werden.

10-Ring-Lactone der Formeln

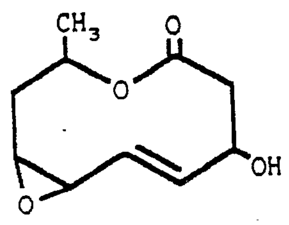

und

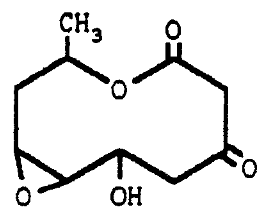

wurden bereits in der Europäischen Patentanmeldung 89104160.0 vorgeschlagen. Sie zeigen antibakterielle Wirkung. Diese Verbindungen werden durch Kultivierung von Mikroorganismen der Spezies Penicillium, insbesondere Penicillium Sp. DSM 4209 und DSM 4210, in einem Nährmedium erhalten.

Es wurde nun gefunden, daß bestimmte Derivate dieser 10-Ring-Lactone die Cholesterin-Biosynthese wirkungsvoll hemmen. Die Erfindung betrifft somit:

10-Ring-Lactone der Formeln I und II

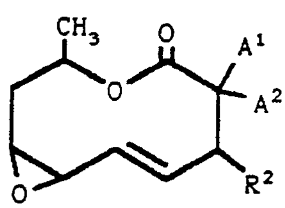

(I)

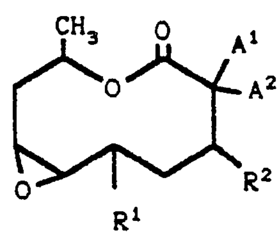

(II)

in denen

$A^1$ und $A^2$

gleich oder verschieden sind und H oder $C_1$-$C_4$-Alkyl bedeuten, wobei der $C_1$-$C_4$-Alkylrest unsubstituiert ist oder ein- oder mehrfach substituiert ist mit OH oder Phenyl, wobei der Phenylrest seinerseits unsubstituiert ist oder ein- oder mehrfach substituiert ist mit Halogen, $CF_3$ oder $C_1$-$C_3$-Alkyl

oder worin

$A^1$ und $A^2$ zusammen einen Rest der Formel

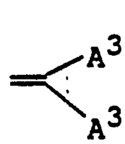

bilden, worin

$A^3$ und $A^{3'}$ gleich oder verschieden sind und $C_1$-$C_2$-Alkoxycarbonyl, Phenyl oder $C_1$-$C_4$-Alkyl bedeuten,

oder worin

$A^1$ und $A^2$

zusammen einen Rest der Formel

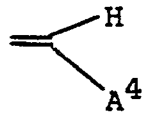 oder 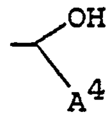

darstellen,
worin $A^4$ Phenyl oder $C_1$-$C_6$-Alkyl bedeutet,
$R^1$
-H, =O oder -$OR^3$ bedeutet und
$R^2$
=O, -$OR^4$, =N-$R^5$ oder

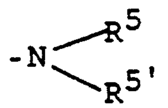

bedeutet, wobei im Falle der Formel II für $R^2$ = =N-$R^5$ und $A^1$ = H diese auch als Verbindung der Formel II'

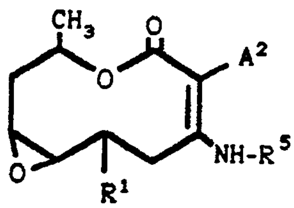

(II')

vorliegen kann
wobei
$R^3$
H, $C_1$-$C_8$-Alkyl, Benzyl, Allyl, MEM, MOM, SEM, THP, 4'-Methoxytetrahydropyran-4'-yl, Trimethylsilyl, Triethylsilyl, Dimethylphenylsilyl, Dimethylcyclohexylsilyl, Dimethyltertiärbutylsilyl oder einen Sulfonsäureester der Formel -$SO_2R^7$ bedeutet, wobei $R^7$ $C_1$-$C_{10}$-Alkyl, Phenyl oder p-Methylphenyl bedeutet
oder
$R^3$
ein Mono- oder Disaccharid darstellt, dessen OH-Gruppen ungeschützt sind oder mit Schutzgruppen geschützt sind
oder
$R^3$
einen Rest der Formel

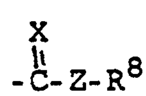 oder 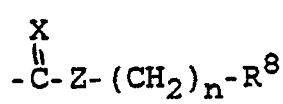

darstellt, wobei
X O oder S

Z O, $-\overset{|}{N}-R^8$, $-\overset{|}{N}-(CH_2)_n-R^8$ oder -NH

n 1 bis 3 bedeutet und
$R^8$ $C_1$-$C_8$-Alkyl, $C_3$-$C_9$-Cycloalkyl, Aryl, Pyridyl, Pyrimidyl oder Pyrazinyl bedeutet
oder
$R^3$

einen Rest der Formel

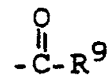

darstellt,
wobei
$R^9$
$C_1$-$C_{20}$-Alkyl, $C_2$-$C_{16}$-Alkenyl mit 1-3 Doppelbindungen,
$C_2$-$C_{16}$-Alkinyl mit 1-3 Dreifachbindungen,
$C_3$-$C_9$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Aryl, Aryloxy, Heteroaryl, Heteroaryloxy, $NHCOCH_3$, $COA^5$, $SO_3A^5$, wobei $A^5$ $C_1$-$C_4$-Alkyl oder H bedeutet
und wobei
alle genannten Aryl-, Heteroaryl-, Pyridyl-, Pyrimidyl- und Pyrazinylreste unsubstituiert sind oder ein- oder mehrfach substituiert sind mit
Halogen, Nitro, Cyano, Hydroxy, Trifluormethyl, Amino, Carboxy, $C_1$-$C_4$-Alkyl-carbonyl, $C_1$-$C_4$-Alkoxy-carbonyl, $C_1$-$C_{10}$-Alkyl, $C_1$-$C_4$-Alkoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff, $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$,
sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H.

Bevorzugt sind 10-Ring-Lactone der Formeln I und II wie oben angegeben, in denen
$A^1$ und $A^2$
gleich oder verschieden sind und H oder $C_1$-$C_2$-Alkyl bedeuten, wobei der $C_1$-$C_2$-Alkylrest unsubstituiert ist oder einfach substituiert ist mit Phenyl, wobei der Phenylrest seinerseits unsubstituiert ist oder einfach substituiert ist mit Halogen, $CF_3$ oder $C_1$-$C_3$-Alkyl
oder worin
$A^1$ und $A^2$
zusammen einen Rest der Formel

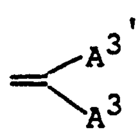

bilden, worin $A^3$ und $A^{3'}$ gleich oder verschieden sind und $C_1$-$C_2$-Alkoxycarbonyl oder Phenyl bedeuten
oder worin
$A^1$ und $A^2$
zusammen einen Rest der Formel

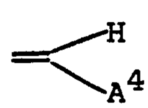

darstellen, worin $A^4$ Phenyl bedeutet,

$R^1$ -H, =O oder $-OR^3$ bedeutet und
$R^2$ =O, $-OR^4$, $=N-R^5$ oder $-N(R^5)(R^{5'})$

bedeutet, wobei im Falle der Formel II für $R^2$ = $=N-R^5$ und $A^1$ = H diese auch als Verbindung der Formel II', wie oben angegeben, vorliegen kann,
wobei
$R^3$
H, $C_1$-$C_8$-Alkyl, Benzyl, Allyl, MEM, MOM, SEM, THP, 4'-Methoxytetrahydropyran-4'-yl, Trimethylsilyl, Triethylsilyl, Dimethylphenylsilyl, Dimethylcyclohexylsilyl, Dimethyltertiärbutylsilyl oder einen Sulfonsäureester der Formel $-SO_2R^7$ bedeutet, wobei
$R^7$ Methyl, Phenyl oder p-Methylphenyl bedeutet
oder
$R^3$
einen Pyranosylrest darstellt, dessen OH-Gruppen ungeschützt sind oder mit Schutzgruppen geschützt sind
oder
$R^3$
einen Rest der Formel

$$-C(=X)-Z-R^8 \quad \text{oder} \quad -C(=X)-Z-(CH_2)_n-R^8$$

darstellt, wobei
X O,

Z O, $-N(-)-R^8$, $-N(-)-(CH_2)_n-R^8$ oder $-NH(-)$

n 1 bis 3 bedeutet und
$R^8$ $C_1$-$C_8$-Alkyl, $C_3$-$C_6$-Cycloalkyl oder Aryl bedeutet oder
$R^3$
einen Rest der Formel

$$-C(=O)-R^9$$

darstellt,
wobei
$R^9$
$C_1$-$C_{15}$-Alkyl, $C_2$-$C_{16}$-Alkenyl, $C_3$-$C_6$-Alkinyl, $C_3$-$C_6$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Phenyl, Phenyloxy, $NHCOCH_3$, $COA^5$, $SO_3A^5$, wobei $A^5$ $C_1$-$C_4$-Alkyl oder H bedeutet
und wobei
alle genannten Aryl- und Heteroarylreste unsubstituiert sind oder ein- oder mehrfach substituiert sind mit Halogen, Cyano, Trifluormethyl, Carboxy, $C_1$-$C_4$-Alkyl-carbonyl, $C_1$-$C_4$-Alkoxy-carbonyl, $C_1$-$C_7$-Alkyl, $C_1$-$C_4$-Alkoxy, $NHCOCH_3$
und worin
$R^4$
unabhängig von $R^3$ die oben für $R^3$ angegebenen Bedeutungen hat und
$R^5$ und $R^{5'}$

gleich oder verschieden sind und Wasserstoff, $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$,
sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H.

Besonders bevorzugt sind 10-Ring-Lactone der Formel I und II wie oben angegeben, in denen
$A^1$ und $A^2$
gleich oder verschieden sind und H oder Methyl bedeuten, wobei der Methylrest unsubstituiert ist oder einfach substituiert ist mit Phenyl,
oder worin
$A^1$ und $A^2$
zusammen einen Rest der Formel

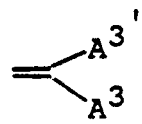

bilden, wobei
$A^3$ und $A^{3'}$ gleich sind und Äthoxycarbonyl bedeuten
$R^1$
-H, =O oder $-OR^3$ bedeutet und
$R^2$
=O, $-OR^4$, $=N-R^5$ oder

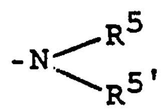

bedeutet, wobei im Falle der Formel II für $R^2$ = $=N-R^5$ und $A^1$ = H diese auch als Verbindung der Formel II', wie oben angegeben, vorliegen kann wobei
$R^3$
H, Methyl, Benzyl, THP, Trimethylsilyl, Dimethyltertiärbutylsilyl oder einen Sulfonsäureester der Formel $-SO_2R^7$ bedeutet, wobei
$R^7$ p-Methylphenyl bedeutet
oder
$R^3$
einen Glucosyl-, Galactosyl- oder Lactosylrest darstellt, dessen OH-Gruppen ungeschützt sind oder mit Acetylgruppen oder Benzylgruppen geschützt sind, wobei diese genannten Zuckerreste entweder glykosidisch gebunden sind oder über einen Orthoester verknüpft sind
oder
$R^3$
einen Rest der Formel

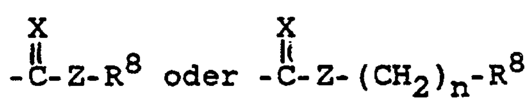

darstellt, wobei
X O,

Z -NH

n 1 bedeutet und
$R^8$ $C_4$-$C_8$-Alkyl oder Aryl bedeutet
oder
$R^3$
einen Rest der Formel

$$-\overset{\overset{\displaystyle O}{\|}}{C}-R^9$$

darstellt,
wobei
$R^9$
$C_1$-$C_{15}$-Alkyl, $C_2$-$C_8$-Alkenyl, $C_3$-$C_6$-Cycloalkyl, Phenyl oder Thienyl oder Furyl bedeutet, wobei die Alkyl-, Alkenyl-, und Cycloalkylreste unsubstituiert sind oder ein- oder mehrfach substituiert sind mit $COOA^5$, F, Cl, Br, Phenyl, Phenyloxy, $NHCOCH_3$, $COA^5$, wobei $A^5$ Methyl oder H bedeutet
und wobei
alle genannten Aryl- oder Phenylreste unsubstituiert sind oder ein- oder mehrfach substituiert sind mit
F, Cl, Br, Trifluormethyl, Carboxy, $C_1$-$C_4$-Alkyl-carbonyl, $C_1$-$C_4$-Alkoxy-carbonyl, $C_1$-$C_7$-Alkyl, Methoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff, $C_1$-$C_6$-Alkyl, Benzyl, Phenyl oder Phenylethyl bedeuten, wobei die genannten Reste unsubstituiert sind oder substituiert sind mit F, Cl, Br oder $OCH_3$,
sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H.

Unter Aryl werden aromatische Kohlenwasserstoffe, insbesondere Phenyl und Naphthyl verstanden und unter Heteroaryl, heteroaromatische Kohlenwasserstoffe, insbesondere Thiophen und Furan, aber auch Pyridin, Pyrimidin und Pyrazin.

Unter Halogenen werden Fluor, Chlor, Brom und Jod verstanden, unter THP Tetrahydropyranyl, unter MEM Methoxyethoxymethyl, unter MOM Methoxymethyl und unter SEM β-Trimethylsilylethoxymethyl.

Alle genannten Alkyl- und Alkenylreste mit mehr als 2 C-Atomen und alle Alkinylreste mit mehr als 3 C-Atomen können sowohl geradkettig als auch verzweigt sein. Bei den genannten Alkenyl- und Alkinylresten handelt es sich sowohl um einfach als auch um mehrfach ungesättigte Verbindungen, bevorzugt um einfach ungesättigte.

Bei den genannten mehrfach substituierten Aryl-, Heteroaryl-, Pyridyl-, Pyrimidyl- und Pyrazinylresten handelt es sich um 2-, 3- oder sofern möglich auch um 4- oder 5-fach substituierte Reste, wobei 2 oder mehrere Substituenten gleich oder verschieden sein können.

Bei den genannten mehrfach substituenten Alkyl-, Alkenyl-, Alkinyl- und Cycloalkylresten können zwei bis alle ersetzbaren Wasserstoffatome durch die genannten Substituenten ersetzt sein. Bevorzugt handelt es sich bei den mehrfach substituierten oben genannten Resten um 2- oder 3-fach substituierte Reste. Die Substituenten können gleich oder verschieden sein.

Bei den genannten Zuckerresten handelt es sich um Mono- und Disaccharide wie sie beispielsweise angegeben sind im "Biochemischen Taschenbuch", 2. Auflage, 1. Teil, Seiten 107-177, Springer-Verlag, Berlin, Göttingen, Heidelberg, 1964. Insbesondere handelt es sich um Hexopyranoside und Hexofuranoside; ganz besonders bevorzugt sind Glucose, Fructose, Allose, Altrose, Mannose, Lactose und Galaktose. Die in den genannten Zuckerresten vorhandenen OH-Gruppen können gegebenenfalls mit in der Zuckerchemie üblichen Schutzgruppen wie Acetyl, Benzoyl, Benzyl oder Acetonyl geschützt sein. Der Zuckerrest kann glykosidisch oder über einen Orthoester gebunden sein.

Weiterhin betrifft die Erfindung ein Verfahren zur Herstellung der Verbindungen der Formeln I und II, wobei man

a) die Verbindung der Formel III

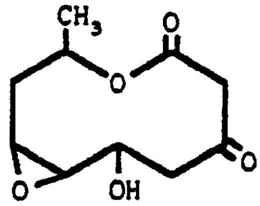 (III)

umsetzt mit einer Verbindung der Formel IV

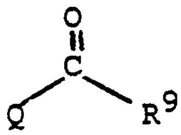 (IV)

worin
Q
OH, Chlorid, Bromid, Imidazolid oder ein Säureanhydrid bedeutet und
$R^9$
die oben zu Formel I und II angegebene Bedeutungen hat,

oder daß man

b) die Verbindung der Formel III umsetzt mit einer Verbindung ausgewählt aus:
Dihydropyran, 4-Methoxy-5,6-dihydropyran, $C_1$-$C_8$-Alkyl-, Benzyl-, Allylchlorid, -bromid, -jodid oder MEM-, MOM-, SEM-chlorid, Trimethylsilyl-, Triethylsilyl-, Dimethylphenylsilyl-, Dimethylcyclohexylsilyl-, Dimethyltertiärbutylsilylchlorid, Sulfonsäurehalogeniden der Formel Hal-$SO_2R^7$, wobei Hal Chlor, Brom oder Jod bedeutet und $R^7$ die oben angegebenen Bedeutungen hat, Isocyanaten der Formel
O = C = N-$R^8$ oder
O = C = N-$(CH_2)_n$-$R^8$, Isothiocyanaten der Formel
S = C = N-$R^8$ oder
S = C = N-$(CH_2)_n$-$R^8$, Carbaminsäurehalogeniden der Formel
Hal'-CO-N-$(R^8)_2$ oder
Hal'-CO-N-$[(CH_2)_n$-$R^8]_2$, Thiocarbaminsäurehalogeniden der Formel Hal'-CS-N-$(R^8)_2$ oder
Hal'-CS-N-$[(CH_2)_n$-$R^8]_2$, oder Verbindungen der Formeln
Hal'-C(O)-O-$R^8$, Hal'-CO-$(CH_2)_n$-$R^8$, Hal'-CS-O-$R^8$ oder
Hal'-CS-O-$(CH_2)_n$-$R^8$
wobei n und $R^8$ die oben zu Formel I und II angegebenen Bedeutungen haben und Hal' Chlor oder Brom bedeutet,

oder daß man

c) die Verbindung der Formel III umsetzt mit einer Verbindung der Formel V

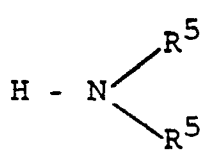 (V)

worin $R^5$ und $R^{5'}$ wie oben zu Formel I und II angegeben definiert sind, und die entstehende C-C-Doppelbindung in der Verbindung der Formel II' gegebenenfalls hydriert,

oder daß man

d) die Verbindung der Formel III umsetzt mit einer Verbindung der Formel VI

$R^3$ - Hal (VI)

wobei $R^3$ ein Mono- oder Disaccharid darstellt und Hal wie oben definiert ist,

oder daß man

e) die Verbindung der Formel III zu einer Verbindung der Formel VII oxidiert

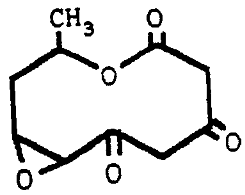

(VII)

oder daß man

f) die Verbindung der Formel III zu einer Verbindung der Formel

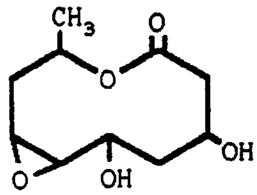

reduziert,

oder daß man

g) eine Verbindung der Formel III zum Enon

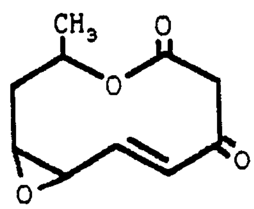

eliminiert und gegebenenfalls die C-C-Doppelbindung hydriert, wobei eine Verbindung der Formel

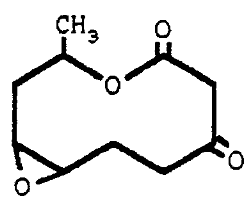

entsteht,

oder daß man

h) die Verbindung der Formel III umsetzt mit einer Verbindung der Formel VIII und/oder VIII'

$A^1$ - Abg (VIII)

$A^2$ - Abg (VIII')

worin $A^1$ und $A^2$ bis auf die Bedeutung

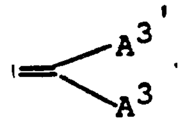

wie in Formel I und II definiert sind und Abg Chlor, Brom, Jod, Sulfat, Mesylat oder Tosylat bedeutet oder daß man

i) die Verbindung der Formel III umsetzt mit einer Verbindung der Formel IX

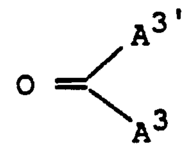

(IX)

worin $A^3$ und $A^{3'}$ wie oben zu Formel I und II definiert sind,

oder daß man

j) die Verbindung der Formel III umsetzt mit einer Verbindung der Formel X,

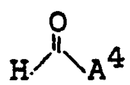

(X)

worin $A^4$ wie oben zu Formel I und II definiert ist

oder daß man

k) die Verbindung der Formel III umsetzt mit Diketen wobei eine Verbindung der Formel II entsteht, mit

$R^1$ = O-C(O)-$CH_2$-C(O)-$CH_3$

oder daß man

l) eine Verbindung der Formel III mit einem Mesoxalsäurediester umsetzt, wobei eine Verbindung der Formel II ensteht mit

$A^1$ und $A^2$ = 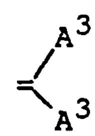 ,

wobei $A^3$ und $A^{3'}$ wie in Formel I und II definiert sind,

oder daß man

zwei oder mehrere der vorstehend genannten Verfahrensvarianten a-1 hintereinander ausführt und daß man anschließend gegebenenfalls die Verbindungen I oder II in ihre physiologisch verträglichen Salze überführt.

Die zur Herstellung von Verbindungen der Formel I notwendige Verbindung der Formel III kann beispielsweise nach dem in der deutschen Patentanmeldung P 3808492.9 - auf die an dieser Stelle ausdrücklich Bezug genommen wird - vorgeschlagenen Verfahren hergestellt werden. Dabei wird die Verbindung der Formel III in einer Nährlösung, die eine Kohlenstoffquelle und eine Stickstoffquelle sowie übliche anorganische Salze enthält, von Penicillium Spec. bevorzugt DSM 4209 oder DSM 4210 produziert. Anstelle der Stämme DSM 4209 oder DSM 4210 können natürlich auch deren Mutanten und Varianten eingesetzt werden, soweit sie diese Verbindung synthetisieren.

Die Bildung der Verbindung der Formel III verläuft besonders gut in einer Nährlösung, die etwa 0,2 bis 5 %, bevorzugt 1 bis 4 %, Malzextrakt, 0,02 bis 0,5 %, bevorzugt 0,1 bis 0,4 % Hefeextrakt, 0,1 bis 5 %, bevorzugt 0,5 bis 2 % Glucose und 0,005 bis 0,2 %, bevorzugt 0,01 bis 0,1 % Ammoniumsalz enthält, jeweils bezogen auf das Gewicht der gesamten Nährlösung. Die Kultivierung erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35° C, vorzugsweise bei etwa 25 bis 30° C, insbesondere bei 27 bis 28° C durchgeführt werden. Der pH-Bereich sollte zwischen 6 und 8 liegen, vorteilhaft zwischen 6,5 und 7,5. Man kultiviert den Mikroorganismus unter diesen Bedingungen im allgemeinen über einen Zeitraum von 60 bis 170 Stunden, bevorzugt 100 bis 150 Stunden.

Zur Isolierung der Verbindung werden Kulturbrühe und Mycel zuerst mit organischen Lösungsmitteln,

wie z. B. Chloroform, Essigester usw. extrahiert, um die unpolaren Verunreinigungen zu entfernen. Anschließend wird mit einem polaren Lösungsmittel, beispielsweise niederen Alkanolen oder Gemischen aus Chloroform und/oder Essigester mit einem niederen Alkanol, extrahiert.

Die Reinisolierung erfolgt vorzugsweise an geeigneten Medien, wie z. B. Kieselgel, Aluminiumoxid oder Ionenaustauschern, durch anschließende Elution mit organischen, polaren Lösungsmitteln oder Lösungsmittelgemischen.

Im folgenden werden die Verfahren a) bis l), die es ermöglichen die unterschiedlich substituierten 10-Ring-Lactone herzustellen, näher beschrieben.

Bei der Verfahrensvarianten a) geht man am besten so vor, daß man die Verbindung der Formel III in äquimolaren Mengen oder in bis zu einem 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel IV bis zur Beendigung der Reaktion umsetzt, gegebenenfalls in Gegenwart einer Base, vorzugsweise Pyridin oder Triethylamin. Setzt man eine Verbindung der Formel IV mit Q = OH ein, so empfiehlt sich die Zugabe von Dicyclohexylcarbodiimid, gegebenenfalls in Anwesenheit eines Katalysators wie DMAP. Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und +40°C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden (DC-Kontrolle).

Die Ausgangsverbindungen für die Verfahrensvariante a), die Verbindungen der Formel IV sind, sofern nicht käuflich, auf einfache Weise nach literaturbekannten Verfahren herstellbar. Beispielsweise erhält man die Säurechloride durch Umsetzung der entsprechenden Carbonsäure mit Thionylchlorid, $PCl_3$ oder $PCl_5$.

Bei der Verfahrensvarianten b) geht man am besten so vor, daß man die Verbindung der Formel III in äquimolaren Mengen oder in einem bis zu 50-fachen Überschuß umsetzt, mit einer Verbindung, die ausgewählt wird aus: Dihydropyran, 4-Methoxy-5,6-dihydropyran, $C_1$-$C_8$-Alkyl-, Benzyl-, Allylchlorid, -bromid, -jodid oder MEM-, MOM-, SEM-Chlorid, Trimethylsilyl-, Triethylslyl-, Dimethylphenylsilyl-, Dimethylcyclohexylsilyl-, Dimethyltert.-butylsilyl-chlorid, Sulfonsäurehalogeniden der Formel Hal-$SO_2R^7$, Isocyanaten der Formel $O=C=N-R^8$ oder $O=C=N-(CH_2)_n-R^8$, Isothiocyanaten der Formel $S=C-N-R^8$ oder $S=C=N-(CH_2)_n-R^8$, Cabaminsäurehalogeniden der Formel Hal'-CO-N-$(R^8)_2$ oder Hal'-CO-N$[(CH_2)_n$- $R^8]_2$, Thiocarbaminsäurehalogeniden der Formel Hal'-CS-N-$(R^8)_2$ oder Hal'CS-N-$[(CH_2)_n-R^8]_2$ oder Verbindungen der Formeln Hal'-C(O)-O-$R^8$, Hal'-CO-$(CH_2)_n-R^8$, Hal'-CS-O-$R^8$ oder Hal'-CS-O-$(CH_2)_n-R^8$.

Gegebenenfalls kann auch diese Umsetzung unter Basenzusatz erfolgen. Als Basen kommen beispielsweise Triethylamin, Pyridin oder Lutidin in Frage. Im Fall der Umsetzung mit Dihydropyran und 4-Methoxy-5,6-dihydropyran können saure Katalysatoren wie $H_2SO_4$, HCl, p-Toloulsulfonsäure oder Pyridinium-p-toloulsulfonat eingesetzt werden. Eine Variante des Verfahrens b) besteht darin, daß man in einem geeigneten, vorzugsweise inerten Lösungsmittel wie Chloroform, Methylenchlorid, THF, Essigester oder Dioxan arbeitet. Auch hier kann der Überschuß der oben aufgeführten Verbindungen bis zur 50-fachen Menge betragen.

Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und -40°C. Die Reaktionszeiten betragen 1 bis 180 Stunden, bevorzugt 1 bis 48 Stunden, besonders bevorzugt 1 bis 8 Stunden. Die Beendigung die Reaktion kann beispielsweise mittels DC-Kontrolle bestimmt werden.

Die Ausgangsverbindungen für die Verfahrensvarianten b) sind, sofern nicht käuflich, auf einfache Weise nach literaturbekannten Verfahren herstellbar. Beispielsweise erhält man die Sulfonsäurehalogenide der Formel Hal-$SO_2R^7$ durch radikalische Umsetzung von Alkanen mit Chlor und $SO_2$ oder durch Halogenierung von Aromaten mit Halogensulfonsäure Hal-$SO_3H$. Die Isocyanate, Isothiocyanate, Carbaminsäurehalogenide und Thiocarbaminsäurehalogenide erhält man nach literaturbekannten Verfahren.

Bei der Verfahrensvarianten c) geht man am besten so vor, daß man die Verbindung der Formel III in äquimolaren Mengen oder in bis zu einem 50-fachen Überschuß, gegebenenfalls in einem inerten Lösungsmittel wie Chloroform, Methylenchlorid, Tetrahydrofuran (THF), Toluol oder Hexan, mit einer Verbindung der Formel V bis zur Beendigung der Reaktion umsetzt, gegebenenfalls in Gegenwart eines Katalysators, vorzugsweise Pyridinium-p-toluolsulfonat oder p-Toluolsulfonsäure, gegebenenfalls in Anwesenheit eines wasserentziehenden Mittels wie Molekularsieb oder durch Azeotropdestillation.

Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesondere zwischen -70°C und +40°C. Die Reaktionszeiten betragen 1 bis 180 Stunden, bevorzugt 1 bis 48 Stunden, besonders bevorzugt 1 bis 12 Stunden.

Die Beendigung der Reaktion kann beispielsweise mittels Dünnschichtchromatographie bestimmt werden.

Die Ausgangsverbindungen für die Verfahrensvariante c), die Verbindungen der Formel V sind, sofern nicht käuflich, auf einfache Weise nach literaturbekannten Verfahren herstellbar.

Bei der Verfahrensvarianten d) geht man am besten so vor, daß man die Verbindung der Formel III in äquimolaren Mengen oder in bis zu einem 50-fachen Überschuß, gegebenenfalls in einem inerten Lösungsmittel wie Chloroform, Methylenchlorid, Nitromethan, Acetonitril oder Toluol, mit einer Verbindung der Formel VI bis zur Beendigung der Reaktion in Anwesenheit eines Katalysators wie Tetraethylammoniumbromid/Molsieb 4Å, $Hg(CN)_2$, $Ag_2CO_3$, $AgClO_4$ oder Silbertrifluormethansulfonat umsetzt, gegebenenfalls in Gegenwart einer Base, wie Collidin, Lutidin oder Tetramethylharnstoff.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante d), die Verbindungen der Formel VI sind, sofern nicht käuflich, auf einfache Weise nach literaturbekannten Verfahren herstellbar. Bevorzugt werden in 2-Position halogensubstituierte Mono- oder Disaccharide.

Bei der Verfahrensvarianten e) geht man am besten so vor, daß man die Verbindung der Formel III in äquimolaren Mengen oder in bis zu einem 50-fachen Überschuß, gegebenenfalls in einem inerten Lösungsmittel wie Chloroform, Tetrachlorkohlenstoff, Methylenchlorid oder Hexan mit einem Oxidationsmittel wie Pyridiniumchlorochromat oder Pyridiniumdichromat bis zur Beendigung der Reaktion umsetzt.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Oxidationsmittel für die Verfahrensvariante e) sind käuflich.

Bei der Verfahrensvarianten f) geht man am besten so vor, daß man die Verbindung der Formel III in äquimolaren Mengen oder in bis zu einem 50-fachen Überschuß, gegebenenfalls in einem inerten Lösungsmittel wie Diethyläther, THF oder niederen Alkoholen mit einem Reduktionsmittel wie Natriumborhydrid, Natriumcyanoborhydrid oder Lithiumaluminiumhydrid bis zur Beendigung der Reaktion umsetzt.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Reduktionsmittel für die Verfahrensvariante f) sind käuflich.

Bei der Verfahrensvarianten g) wird die Verbindung der Formel III zunächst nach dem Verfahren b) in ein entsprechendes O-Mesylat oder O-Tosylat überführt und dieses dann mittels Base nach literaturbekannten Verfahren eliminiert. Das Eliminierungsprodukt kann dann nach literaturbekannten Verfahren hydriert werden. Hierbei kommen die gängigen Hydrierkatalysatoren wie Palladium- oder Platinkatalysatoren zur Anwendung.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Bei der Verfahrensvarianten h) geht man am besten so vor, daß man die Verbindung der Formel III in äquimolaren Mengen oder in bis zu einem 50-fachen Überschuß, gegebenenfalls in einem inerten Lösungsmittel wie Chloroform, Methylenchlorid, Tetrahydrofuran (THF), Essigester oder Dioxan, mit einer Verbindung der Formel VIII und/oder VIII' bis zur Beendigung der Reaktion umsetzt.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante h), die Verbindungen der Formel VIII bzw. VIII'

sind, sofern nicht käuflich, auf einfache Weise nach literaturbekannten Verfahren herstellbar.

Bei der Verfahrensvarianten i) geht man am besten so vor, daß man die Verbindung der Formel III in äquimolaren Mengen oder in bis zu einem 50-fachen Überschuß, gegebenenfalls in einem inerten, aprotischen Lösungsmittel wie Chloroform, Methylenchlorid, Tetrahydrofuran (THF), Essigester oder Dioxan, mit einer Verbindung der Formel IX bis zur Beendigung der Reaktion umsetzt, gegebenenfalls in Gegenwart einer Base wie Pyridin oder Piperidin.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante i), die Verbindungen der Formel IX sind, sofern nicht käuflich, auf einfache Weise nach literaturbekannten Verfahren herstellbar.

Bei der Verfahrensvarianten j) geht man am besten so vor, daß man die Verbindung der Formel III in äquimolaren Mengen oder in bis zu einem 50-fachen Überschuß, gegebenenfalls in einem inerten Lösungsmittel wie Tetrahydrofuran (THF) oder Diethylether mit einer Verbindung der Formel X bis zur Beendigung der Reaktion umsetzt, gegebenenfalls in Gegenwart einer Base, vorzugsweise Pyridin oder Piperidin.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante j), die Verbindungen der Formel X sind, sofern nicht käuflich, auf einfache Weise nach literaturbekannten Verfahren herstellbar.

Bei der Verfahrensvarianten k) geht man am besten so vor, daß man die Verbindung der Formel III in äquimolaren Mengen oder in bis zu einem 50-fachen Überschuß, gegebenenfalls in einem inerten Lösungsmittel wie Chloroform, Methylenchlorid Tetrahydrofuran (THF), Essigester oder Dioxan, mit Diketen bis zur Beendigung der Reaktion umsetzt, gegebenenfalls in Gegenwart einer Base wie Pyridin oder Triethylamin.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindung Diketen für die Verfahrensvariante k), ist käuflich.

Bei der Verfahrensvariante l) geht man am besten so vor, daß man die Verbindung der Formel III in äquimolaren Mengen oder in bis zu einen 50-fachen Überschuß, gegebenenfalls in einem inerten Lösungsmittel wie Chloroform, Methylenchlorid, Tetrahydrofuran (THF), Essigester oder Dioxan und gegebenenfalls unter Zusatz einer Base wie Pyridin oder Piperidin, mit einem Mesoxalsäurediester bis zur Beendigung der Reaktion umsetzt.

Die Reaktionstemperaturen liegen dabei zwischen -70° C und +100° C, vorzugsweise bei Verwendung eines Lösungsmittels zwischen dem Festpunkt und dem Siedepunkt des Lösungsmittels, insbesondere zwischen -70° C und +40° C. Die Reaktionszeiten betragen 1 bis 180 Stunden, bevorzugt 1 bis 48 Stunden, besonders bevorzugt 1 bis 8 Stunden. Die Beendigung der Reaktion kann beispielsweise mittels Dünnschichtchromatographie bestimmt werden.

Die Ausgangsverbindungen für die Verfahrensvariante l), die Mesoxalsäurediester sind, sofern nicht käuflich, auf einfache Weise nach literaturbekannten Verfahren herstellbar.

Als Ausgangssubstanz für die Umsetzungen gemäß der Verfahrensvarianten a)-l) kann nicht nur die Verbindung der Formel III verwendet werden, sondern auch eine bereits nach einem oder mehreren der Verfahren a)-l) derivatisierte Verbindung. Auf diese Weise lassen sich nacheinander die verschiedensten Substituenten $A^1$, $A^2$, $R^1$ und/oder $R^2$ in das Grundgerüst der Formeln I oder II einführen.

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol oder Chloroform, Dichlormethan oder Essigester oder Methanol/Chloroform-Mischungen oder Chloroform/Hexan/Methanol-Mischungen aber auch durch extraktive Methoden wie flüssig/flüssig-Extraktion oder fest/flüssig-Extraktion oder durch Kristallisation gereinigt werden.

Die Inhibierung der Cholesterin-Biosynthese durch die erfindungsgemäßen Verbindungen sowie deren physiologisch verträgliche Salze und einschließlich der Verbindung der Formel I in der $R^2$-$OR^4$ bedeutet mit

$R^4$ = H, sowie einschließlich der Verbindung der Formel II, in der $R^1$-$OR^3$ und $R^2$ = O Bedeutet, mit $R^3$ = H wurde in in-vitro- und in-vivo-Tests überprüft. Dabei zeigte sich, daß die erfindungsgemäßen Derivate eine hervorragende Wirkung als Inhibitoren der Cholesterin-Biosynthese zeigen. Sie können dementsprechend als Lipidsenker eingesetzt werden.

Die erfindungsgemäßen Verbindungen sind aufgrund ihrer pharmakologischen Eigenschaften für die Behandlung von Störungen des Stoffwechsels von Cholesterin und cholesterinähnlichen Stoffen geeignet.

Die Erfindung betrifft daher weiter die Anwendung der erfindungsgemäßen Verbindungen der Formeln I und II sowie deren physiologisch verträglicher Salze bei der Behandlung und Prophylaxe von Störungen des Stoffwechsels von Cholesterin und cholesterinähnlichen Stoffen.

Die Verbindungen können entweder allein oder mit physiologisch verträglichen Hilfs- oder Trägerstoffen vermischt als Arzneimittel angewandt werden. Sie können zu diesem Zweck oral in Dosen von 0,01 - 5,0 mg/kg/Tag, vorzugsweise 0,01 - 1,0 mg/kg/Tag oder parenteral subkutan in Dosen von 0,001 - 2,5 mg/kg/Tag, vorzugsweise 0,001 - 1,0 mg/kg/Tag, inbesondere 0,005 - 0,2 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Die Erfindung umfaßt weiterhin die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder II und/oder deren physiologisch verträgliche Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoff) entweder als solche oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 % beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Die Wirkstoffe können oral, parenteral (subkutan) oder rectal appliziert werden.

Die aktiven Verbindungen werden mit den dafür geeigneten Zusatzstoffen wie Trägerstoffen, Stabilisatoren oder inerten Verdünnungsmitteln vermischt und durch die üblichen Methoden in geeignete Darreichungsformen gebracht, wie Tabletten, Dragees, Steckkapseln, wäßrige alkoholische oder ölige Suspensionen oder wäßrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke, verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation werden die aktiven Verbindungen gewünschtenfalls mit den dafür geeigneten Substanzen wie Lösungsvermittler, Emulgatoren oder weiteren Hilfsstoffen in Lösung, Suspension oder Emulsion gebracht. Als Lösungsmittel kommen z. B. in Frage physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Nachfolgend ist die Erfindung an Hand von Beispielen näher erläutert.

Alle nach den folgenden Beispielen erhaltenen Verbindungen wurden mittels $^1$H-NMR und/oder $^{13}$C-NMR und/oder IR und/oder C,H-Analyse und/oder Massenspektrum charakterisiert. Das 10-Ring-Lacton der Formel III wurde gemäß der deutschen Patentanmeldung P 3808492.9 hergestellt.

Herstellung der Verbindung der Formel III

a) Herstellung einer Sporensuspension des Produzentenstammes:

100 ml Nährlösung (2 g Hefeextrakt, 20 g Malzextrakt, 10 g Glucose, 0,5 g $(NH_4)_2PO_4$, 1 l Leitungswasser, pH-Wert vor dem Sterilisieren 7,3) in einem 500 ml Erlenmeyerkolben werden mit dem Stamm DSM 4209 oder DSM 4210 beimpft und 72 Stunden bei 25 °C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigungen zugegeben wurde, gleichmäßig verteilt und dekantiert. Die Kulturen werden 10 bis 14 Tage bei 25 °C inkubiert. Die nach dieser

Zeit entstandenen Sporen eines Kolbens werden mit 500 ml entionisiertem Wasser, das einen Tropfen eines handelsüblichen nichtionischen Tensids (Triton X 100, Fa. Serva) enthält, abgeschwemmt, sofort weiterverwendet oder bei -22° C aufbewahrt.

b) Herstellung einer Kultur bzw. Vorkultur des Produzentenstammes im Erlenmeyerkolben.

Ein 500 ml Erlenmeyerkolben mit 100 ml der unter a) beschriebenen Nährlösung wird mit einer auf einem Schrägröhrchen gezogenen Kultur oder mit 0,2 ml Sporensuspension angeimpft und auf einer Schüttelmaschine bei 120 UpM und 25° C inkubiert. Die maximale Produktion ist nach ca. 120 Stunden erreicht. Zum Animpfen von 10 und 100 l Fermentern genügt eine 48 Stunden alte Submerskultur (5 %) aus der gleichen Nährlösung.

c) Herstellung der Verbindung der Formel III

Ein 10 l Fermenter wird unter folgenden Bedingungen betrieben:

| | |
|---|---|
| Nährmedium: | 20 g/l Malzextrakt |
| | 2 g/l Hefeextrakt |
| | 10 g/l Glucose |
| | 0,5 g/l $(NH_4)_2PO_4$ |
| | pH 7,2 |
| Inkubationszeit: | 150 Stunden |
| Inkubationstemperatur: | 25°C |
| Rührergeschwindigkeit: | 250 UpM |
| Belüftung: | 4 l Luft/min. |

Durch wiederholte Zugabe weniger Tropfen ethanolischer Polyollösung kann die Schaumentwicklung unterdrückt werden. Das Produktionsmaximum wird nach ca. 150 Stunden erreicht. Die Ausbeuten liegen bei ca. 100 mg/l.

d) Isolierung der Verbindung der Formel III

Nach der Fermentation von DSM 4209 bzw. DSM 4210 wird die Kulturbrühe unter Zusatz von 2 % Celite als Filterhilfsmittel filtriert. Die Verbindung der Formel III wird aus dem Kulturüberstand isoliert. Es wird nach den folgendem Schema verfahren:

**Aufarbeitung / Isolierung**

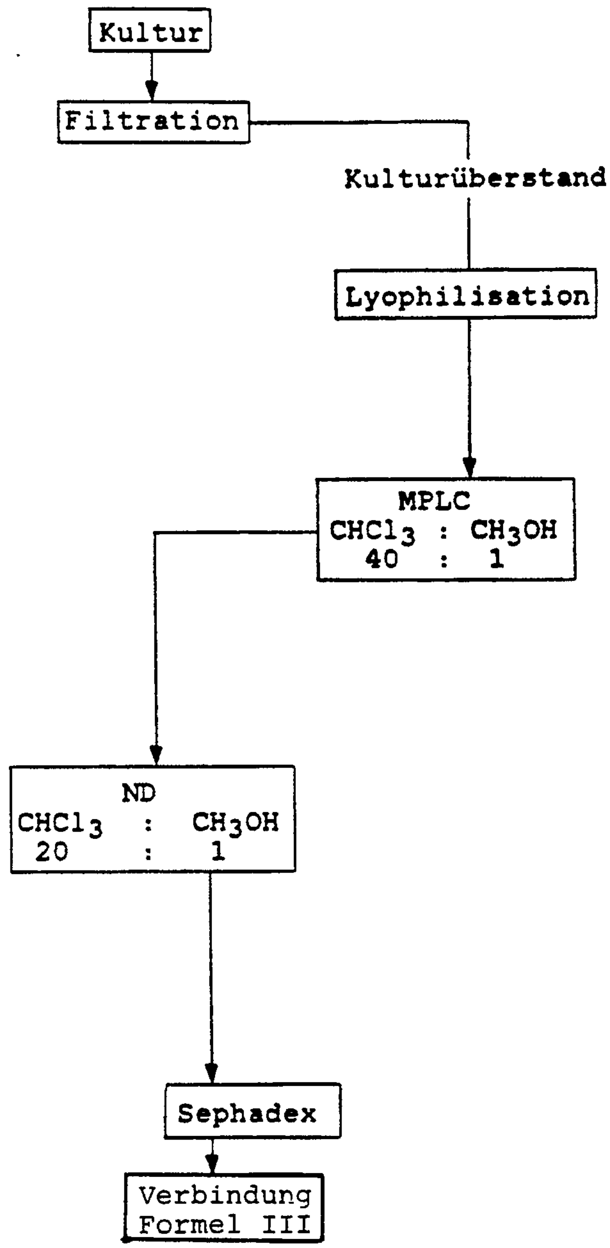


Abkürzungen:
MPLC: Mitteldruck-Flüssigchromatographie
ND: Normaldruck-Säulenchromatographie

**Beispiel 1:**

Darstellung von 6-Butylcarbonyloxy-2,4-dioxo-10-methyl-1-oxa-7-cyclodecenoxid (II.11)

100 mg des Zehnringlactons der Formel III (0,47 mmol) werden in 3 ml Dichlormethan (absolut) gelöst und mit 0,4 ml Valeriansäureanhydrid, 42,5 mg (0,54 mmol) abs. Pyridin (15 % Überschuß) und einer Spatelspitze N,N-Dimethylaminopyridin 18 h bei Raumtemperntur gerührt. Anschließend wird das Lösungsmittel i. Vak. entfernt und das verbleibende Öl durch Flash-Chromatographie an Kieselgel (mobile Phase Chloroform) gereinigt.
Ausbeute: 118,5 mg (0,40 mmol; 85 % d. Th.), farbloses Öl

| | | | |
|---|---|---|---|
| $C_{15}H_{22}O_6$: | gef.: | C 60,3 | H 7,3 |
| | ber.: | C 60,4 | H 7,4 |

**Beispiel 2:**

Darstellung von 2,4-Dioxo-10-methyl-1-oxa-6-(phenyl-amino-carbonyl-oxy)-7-cyclodecenoxid (II.4)

214 mg des Zehnringlactons der Formel III (1 mmol) werden in 2,5 ml absolutem Pyridin gelöst und mit 125 mg (1,05 mmol) Phenylisocyanat 12 h bei Raumtemperatur gerührt. Zur Aufarbeitung wird das Reaktionsgemisch im Vakuum eingeengt, der Rückstand mit Dichlormethan aufgenommen, die organische Phase mit Wasser gewaschen, über $Na_2SO_4$ getrocknet und im Vakuum eingeengt. Das zurückbleibende Öl wird mittels Flash-Chromatographie an Kieselgel (mobile Phase: $CHCl_3$/MeOH = 40/l) gereinigt.
Ausbeute: 166 mg(0,50 mmol; 50 % d. Th.); farblose Kristalle vom Schmelzpunkt 149 °C

| | | | |
|---|---|---|---|
| $C_{17}H_{19}NO_6$: | gef.: | C 61,1 | H 5,6 |
| | ber.: | H 61,3 | H 5,8 |

**Beispiel 3:**

Darstellung von 10-Methyl-1-oxa-2,4,6-trioxo-7-cyclodecenoxid (II.19)

50 mg des Zehnringlactons der Formel III (0,23 mmol) werden in 2,5 ml Dichlormethan gelöst und bei einer Temperatur von 0 °C mit 75,5 mg (1,5facher Überschuß) Pyridiniumchlorochromat ("PCC") versetzt. Das Reaktionsgemisch wird 3 h bei dieser Temperatur gerührt.

Anschließend wird über Filterhilfe filtriert und die organische Phase zweimal mit Wasser gewaschen. Nach Trocknen über $Na_2SO_4$ und Entfernen des Lösungsmittels im Vakuum verbleibt ein gelbes Öl, welches mittels Flash-Chromatographie an Kieselgel (mobile Phase: $CHCl_3$/Hexan/Methanol = 8/8/1) gereinigt wird.
Ausbeute: 26,5 mg (0,13 mmol; 54 % d. Th.); farbloses Öl

| | | | |
|---|---|---|---|
| $C_{10}H_{12}O_5$: | gef.: | C 56,8 | H 5,6 |
| | ber.: | C 56,6 | H 5,7 |

$^{13}C$-NMR-Spektrum (90,5 MHz; $CDCl_3$/TMS):
$\delta$ = 192,53 (C-4); 192,19 (C-6); 164,69 (C-2); 69,10 (C-10); 56,74 (C-7); 56,07 (C-8); 55,98 (C-3); 52,05 (C-5); 33,60 (C-9); 20,57 (C-11)

**Beispiel 4:**

Darstellung von 4-Hexylamino-6-hydroxy-10-methyl-1-oxa-3-cyclodecen-7-enoxid (II'.1)

50 mg des Zehnringlactons der Formel III werden in 2 ml absolutem Tetrahydrofuran gelöst und mit 2,0 Äquivalenten n-Hexylamin (48 mg) versetzt. Das Reaktionsgemisch wird 3 Tage bei Raumtemperatur gerührt.

Zur Aufarbeitung wird im Vakuum vom Lösungsmittel befreit, der verbleibende Rückstand mit Dichlormethan aufgenommen, zweimal mit Wasser gewaschen, über $Na_2SO_4$ getrocknet und im Vakuum eingeengt. Das verbleibende Öl wird durch Chromatographie an Sephadex LH-20-100 (25 - 100 μ; mobile Phase: Methanol) gereinigt.

Ausbeute: 65 mg (0,22 mmol; 94 % d. Th.); farbloses Öl

| | | | |
|---|---|---|---|
| $C_{16}H_{27}NO_4$: | gef.: | C 64,5 | H 9,1 |
| | ber.: | C 64,6 | H 9,2 |

$^{13}C$-NMR-Spektrum (90,5 MHz; $CDCl_3$/TMS):

δ = 169,88 (C-2); 157,51 (C-4); 85,42 (C-3); 70,40 (C-6); 66,22 (C-10); 60,44 (C-7); 55,36 (C-8); 43,56 (C-12); 38,48 (C-5); 37,15 (C-9); 31,35 (C-15); 28,18 (C-13); 26,61 (C-14); 22,44 (C-16); 21,10 (C-11), 13,88 (C-17)

**Beispiel 5:**

Darstellung von 2,4-Dioxo-6-(1,3-dioxo-but-1-yl)-oxy-10-methyl-1-oxa-7-cyclodecenoxid (II.31)

50 mg des Zehnringlactons der Formel III werden in 3 ml absolutem Dichlormethan gelöst und mit 2 μl absolutem Pyridin (10 mol-%) sowie 19 μl Diketen (1,05 Äquivalente) versetzt. Die Reaktionspartner werden 24 h bei Raumtemperatur gerührt.

Zur Aufarbeitung wird die organische Phase mit 50 ml Dichlormethan verdünnt, mehrmals mit Wasser gewaschen, über $Na_2SO_4$ getrocknet und das Lösungsmittel im Vakuum entfernt. Der verbleibende Rückstand wird mittels Flash-Chromatographie an Kieselgel (mobile Phase: $CHCl_3$/Hexan/Methanol = 16/16/1) gereinigt.

Ausbeute: 22,3 mg (0,75 mmol; 32 % d. Th.); farbloses Öl

| | | | |
|---|---|---|---|
| $C_{14}H_{18}O_7$: | gef.: | C 56,2 | H 6,0 |
| | ber.: | C 56,4 | H 6,1 |

**Beispiel 6:**

Glykosilierung des Zehnringlactons der Formel III mittels α-Brom-tetraacetyl-galactose (II.16)

50 mg des Zehnringlactons der Formel III werden in 5 ml $CH_2Cl_2$ gelöst und unter Rühren bei Raumtemperatur mit 170 mg Brom-tetraacetyl-galactose (2 Äquivalente), 150 μl Collidin (5 Äquivalente), 100 mg (ca. 2 Äquivalente) $AgClO_4$ und 3 g fein gepulvertem Molsieb (3Å) versetzt. Das Reaktionsgemisch wird 2 Tage bei Raumtemperatur gerührt.

Zur Aufarbeitung wird das Reaktionsgemisch filtriert, zweimal mit Wasser gewaschen, über $Na_2SO_4$ getrocknet und das Lösungsmittel im Vakuum entfernt. Der Rückstand wird durch Chromatographie an Kieselgel (mobile Phase: $CHCl_3$/Hexan/Methanol = 8/8/1) gereinigt.

Ausbeute: 48 mg (0,88 mmol; 38 % d. Th.); farbloses Öl

(Bei dem Reaktionsprodukt handelt es sich lt. spektroskopischen Daten um den Orthoester.)

| | | | |
|---|---|---|---|
| $C_{24}H_{32}O_{14}$: | gef.: | C 52,7 | H 5,8 |
| | ber.: | C 52,9 | H 5,9 |

$^{13}C$-NMR-Spektrum (90,5 MHz; $CDCl_3$/TMS):
δ = 199,82 (C-4); 165,34 (C-2); 120,93 (C-12); 97,83 (C-14); 73,00 (C-15); 70,72 (C-16); 69,40 (C-6); 68,99 (C-10); 68,75 (C-18); 65,69 (C-17); 61,55 (C-19); 59,51 (C-7); 54,97 (C-8); 52,07 (C-3); 47,92 (C-5); 36,77 (C-9); 24,16 (C-13); 20,64 (C-11); weiterhin 3 Acetylgruppen mit Signalen bei 170,41; 169,96; 169,80; 20,69; 20,64 und 20,48

**Beispiel 7:**

Darstellung von 3,3-Dimethyl-2,4-dioxo-6-methoxy-10-methyl-1-oxa-7-cyclodecenoxid (II.40)

50 mg des Zehnringlactons der Formel III werden in 2 ml absolutem N,N-Dimethylformamid gelöst und bei Raumtemperatur mit 0,5 ml Iodmethan und 400 mg frisch gefälltem Silberoxid versetzt. Das Reaktionsgemisch wird 6 h bei Raumtemperatur gerührt.

Zur Aufarbeitung wird die Reaktionslösung uber Filterhilfe filtriert. Das Lösungsmittel wird anschließend im Ölpumpenvakuum entfernt und der Rückstand durch Chromatographie an Kieselgel (mobile Phase: $CHCl_3$) gereinigt.
Ausbeute: 55 mg (0,22 mmol; 92 % d. Th.); farbloses Öl

| | | | |
|---|---|---|---|
| $C_{13}H_{20}O_5$: | gef.: | C 60,9 | H 7,7 |
| | ber.: | C 60,9 | H 7,9 |

**Beispiel 8:**

Darstellung 2,4-Dioxo-6- (4-fluor-2-chlor-phenyl)-carbonyl-oxy-10-methyl-1-oxa-7-cyclodecenoxid (II.38)

50 mg des Zehnringlactons der Formel III werden in 3 ml absolutem Dichlormethan gelöst und mit 48 mg (1 Äquivalent) Dicyclohexylcarbodiimid, 41 mg (1 Äquivalent) 4-Fluor-2-chlor-benzoesäure, sowie 2 mg N,N-Dimethylaminopyridin versetzt. Das Reaktionsgemisch wird 12 h bei Raumtemperatur gerührt.

Zur Aufarbeitung wird über Filterhilfe filtriert, die organische Phase mit Dichlormethan verdünnt, mit Wasser extrahiert, über $Na_2SO_4$ getrocknet und das Lösungsmittel im Vakuum entfernt. Der Rückstand wird mittels Chromatographie an Kieselgel (mobile Phase: $CHCl_3$) gereinigt.
Ausbeute: 52 mg (0,14 mmol; 60 % d. Th.); farbloses Öl

| | | | |
|---|---|---|---|
| $C_{17}H_{16}ClFO_6$: | gef.: | C 54,8 | H 4,3 |
| | ber.: | C 55,0 | H 4,4 |

**Beispiel 9:**

Darstellung von 2,4-Dioxo-10-methyl-1-oxa-6-trimethylsilyl-oxy-7-cyclodecenoxid (II.9)

30 mg des Zehnringlactons der Formel III (0,14 mmol) werden in 5 ml Dichlormethan gelöst und mit 16,7 mg (1,1 Äquivalenten) Trimethylchlorsilan und 14,4 mg (1,3 Äquivalenten) absolutem Pyridin versetzt. Das Reaktionsgemisch wird 6 h bei Raumtemperatur gerührt.

Zur Aufarbeitung wird mit 50 ml Dichlormethan versetzt, die organische Phase zweimal mit Wasser gewaschen, über $Na_2SO_4$ getrocknet und das Lösungsmittel im Vakuum entfernt. Das Reaktionsprodukt wird mittels Flash-Chromatographie an Kieselgel (mobile Phase: $CHCl_3$) gereinigt.
Ausbeute: 24,9 mg (0,87 mmol; 62 % d. Th.)

| | | | |
|---|---|---|---|
| $C_{13}H_{22}O_5Si$: | gef.: | C 54,7 | H 7,6 |
| | ber.: | C 54,5 | H 7,7 |

**Beispiel 10**

Darstellung von 3-(Bis-ethoxycarbonyl)-methylen-2,4-dioxo-6-hydroxy-10-methyl-1-oxa-7-cyclodecenoxid (II.17)

70 mg des Zehnringlactons der Formel III werden in 3 ml absolutem Chloroform gelöst und bei einer Temperatur von -20° C mit 0,1 ml (2,2 Äquivalenten) Triethylamin und 60 µl (1,2 Äquivalenten) Mesoxalsäurediethylester versetzt. Nach 2 h bei -20° C wird 4 h bei Raumtemperatur gerührt.

Zur Aufarbeitung wird von Lösungsmittel und Base im ölpumpenvakuum befreit und das zurückbleibende hellgelbe Öl mittels Chromatographie an Sephadex LH-20 (mobile Phase: Methanol) gereinigt.
Ausbeute: 101,2 mg (0,27 mmol; 84 % d. Th.); farbloses Öl

| | | | |
|---|---|---|---|
| $C_{17}H_{22}O_9$: | gef.: | C 55,3 | H 5,8 |
| | ber.: | C 55,1 | H 6,0 |

**Beispiel 11:**

Darstellung von 4,6-Dihydroxy-10-methyl-1-oxa-2-oxo-7-cyclodecenoxid (II.2)

50 mg des Zehnringlactons der Formel III (0,234 mmol) werden in 5 ml absolutem Tetrahydrofuran gelöst und bei Raumtemperatur portionsweise mit 16,8 mg (1,15 Moläquivalente) Natriumcyanoborhydrid versetzt. Der pH-Wert der Reaktionslösung wird durch Zugabe einer Lösung von HCl/Eisessig/THF auf pH 4 gehalten (Indikator Methylorange). Nach 4 h wird die organische Phase mit 50 ml Dichlormethan versetzt, mit Wasser zweimal extrahiert, über $Na_2SO_4$ getrocknet und das Lösungsmittel im Vakuum abdestilliert. Der verbleibende Rückstand wird mittels Chromatographie an Kieselgel (mobile Phase: Ethylacetat) gereinigt.
Ausbeute: 23,3 mg (0,11 mmol; 46 % d. Th.)

| | | | |
|---|---|---|---|
| $C_{10}H_{16}O_5$: | gef.: | C 55,3 | H 7,4 |
| | ber.: | C 55,5 | H 7,5 |

**Beispiel 12:**

Darstellung von 2,4-Dioxo-10-methyl-1-oxa-6-tetrahydro-pyranyloxy-7-cyclodecenoxid (II.43)

50 mg des Zehnringlactons der Formel III (0,23 mmol) werden bei Raumtemperatur in 3 ml absolutem Dichlormethan gelöst und mit 29,5 mg (0,35 mmol; 1,5 Moläquivalente) Dihydropyran sowie einer Spatelspitze Pyridinium-p-toluolsulfonat ("PPTS") versetzt. Das Reaktionsgemisch wird 24 h bei Raumtemperatur stehengelassen.

Zur Aufarbeitung wird das Reaktionsgemisch zweimal mit wenig gesättigter Ammoniumchloridlösung gewaschen, über $Na_2SO_4$ getrocknet und das Lösungsmittel im Vakuum entfernt. Die Reinigung des Reaktionsproduktes erfolgt mittels Flash-Chromatographie an Kieselgel (mobile Phase: $CHCl_3$/Hexan/Methanol = 20/20/1).
Ausbeute: 51,8 mg (17,4 mmol; 74 % d. Th.)

$$C_{15}H_{22}O_6: \quad \text{gef.:} \quad C\ 60{,}6 \quad H\ 7{,}5$$
$$\text{ber.:} \quad C\ 60{,}4 \quad H\ 7{,}4$$

(lt. spektroskopischen Daten handelt es sich bei dem Reaktionsprodukt um ein ca. 1:1 Gemisch der beiden diastereomeren Tetrahydropyranylether)

**Beispiel 13:**

Darstellung von 2,4-Dioxo-10-methyl-1-oxa-5-cyclodecen-7-cenoxid (I.1)

50 mg des Zehnringlactons der Formel III (0,23 mmol) werden in 3 ml absolutem Pyridin gelöst und mit 200 µl p-Toluolsulfonsäurechlorid versetzt. Anschließend wird 14 h bei Raumtemperatur gerührt.

Zur Aufarbeitung wird auf 50 ml Dichlormethan gegossen, die organische Phase dreimal mit Wasser gewaschen, über $Na_2SO_4$ getrocknet und das Lösungsmittel im Vakuum entfernt.

Das Reaktionsprodukt wird sodann in 2,5 ml absolutem Toluol gelöst und mit 1 Äquivalent Diazabicycloundecen 3 h bei 80° C gerührt. Anschließend wird nochmals wie oben angegeben aufgearbeitet und das Reaktionsprodukt mittels Flash-Chromatographie gereinigt (Kieselgel; Laufmittel $CHCl_3$).
Ausbeute: 22 mg (0,11 mmol; 48 d. Th); farbloses Öl

$$C_{10}H_{12}O_4: \quad \text{gef.:} \quad C\ 60{,}9 \quad H\ 6{,}2$$
$$\text{ber.:} \quad C\ 61{,}2 \quad H\ 6{,}2$$

**Beispiel 14:**

Darstellung von 2,4-Dioxo-10-methyl-1-oxa-7-cyclodecenoxid (II.44)

20 mg der im Beispiel 13 hergestellten Verbindung werden in 3 ml Tetrahydrofuran gelöst und mit ca. 4 mg Palladium auf Kohle (5 Prozent auf Aktivkohle) als Hydrierkatalysator versetzt und das Gemisch unter kräftigem Rühren 1 h in einer Wasserstoffatmosphäre belassen.

Anschließend wird vom Katalysator über Filterhilfe abfiltriert, das Lösungsmittel im Vakuum abdestilliert und das verbleibende Öl mittels Flash-Chromatographie an Kieselgel (mobile Phase: $CHCl_3$/Hexan/Methanol = 16/16/1) gereinigt.
Ausbeute: 17 mg (0,086 mmol; 92 % d. Th.)

$$C_{10}H_{14}O_4: \quad \text{gef.:} \quad C\ 60{,}3 \quad H\ 7{,}0$$
$$\text{ber.:} \quad C\ 60{,}6 \quad H\ 7{,}1$$

Die nachfolgende Tabelle 1 gibt eine Übersicht über die hergestellten Verbindungen und über solche, die analog zu den Beispielen 1 - 14 hergestellt wurden.

Tabelle 1

| Beisp.-Nr. | Verbindung | $R^1$ | $R^2$ | $A^1$ | $A^2$ | Reagenz | Bedingungen | Verfahren | Ausf.gem. Bsp. Nr. | Ausbeute |
|---|---|---|---|---|---|---|---|---|---|---|
| | II.1 | $OC(O)CH_3$ | =O | H | H | Acetanhydrid/Pyr./DMAP | 12 h/RT | a) | 1 | 75 % |
| 11 | II.2 | OH | OH | H | H | $NaBH_3CN$/pH 4 | 2 h/RT | f) | 11 | 46 % |
| | II.3 | $OC(O)C_6H_5$ | =O | H | H | Benzoesäureanhydrid/Pyr., DMAP (Lsg.: $CHCl_3$) | 12 h/RT | a) | 1 | 74 % |
| 2 | II.4 | $OC(O)NH-C_6H_5$ | =O | H | H | Phenylisocyanat/Pyr. | 12 h/RT | b) | 2 | 50 % |
| | II.5 | $OC(O)-(CH_2)_5-CH_3$ | =O | H | H | $O(C(O)(CH_2)_5CH_3)_2$, Pyr., DMAP, $CH_2Cl_2$ | 18 h/RT | a) | 1 | 66 % |
| | II.6 | $OC(O)NH-(2,4-(OCH_3)_2-C_6H_3)$ | O | H | H | 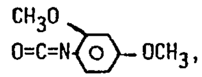, Pyr. | 4 h/RT | b) | 2 | 45 % |
| | II.7 | $OC(O)-NH_2$-octyl | =O | H | H | Octylisocyanat, $NEt_3$, Ether | 12 h/RT | b) | 2 | 52 % |
| | II.8 | $OC(O)C_2H_5$ | =O | H | H | $(CH_3-CH_2-\overset{O}{\overset{\|}{C}})_2O$, Pyr., DMAP, $CH_2Cl_2$ | 18 h/RT | a) | 1 | 81 % |
| 9 | II.9 | $OSi(CH_3)_3$ | =O | H | H | TMSCl, Pyr. | 6 h/RT | b) | 9 | 62 % |
| | II.10 | 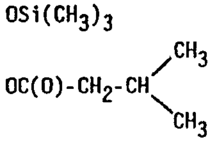 | =O | H | H | 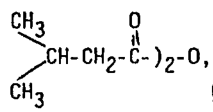, Pyr., DMAP | 18 h/RT | a) | 1 | 72 % |
| 1 | II.11 | $OC(O)-CH_2-CH_2-CH_2-CH_3$ | =O | H | H | $(CH_3(CH_2)_3-\overset{O}{\overset{\|}{C}})_2O$, Pyr., DMAP | 18 h/RT | a) | 1 | 85 % |
| | II.12 | 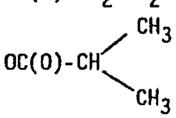 | =O | H | H | 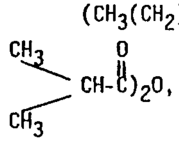, Pyr., DMAP | 12 h/RT | a) | 1 | 68 % |

Fortsetzung Tabelle 1

| Beisp.-Nr. | Verbindung | $R^1$ | $R^2$ | $A^1$ | $A^2$ | Reagenz | Bedingungen | Verfahren | Ausf.gem. Bsp. Nr. | Ausbeute |
|---|---|---|---|---|---|---|---|---|---|---|
| | II.13 | $OC(O)-(CH_2)_4-CH_3$ | =O | H | H | $CH_3(CH_2)_4-\overset{O}{\overset{\|}{C}})_2O$, Pyr. DMAP | 12 h/RT | a) | 1 | 71 % |
| | II.14 | $O-\overset{O}{\overset{\|}{C}}-thien-2-yl$ | =O | H | H | $(thien-2-yl-\overset{O}{\overset{\|}{C}})_2O$, Pyr. DMAP | 18 h/RT | a) | 1 | 59 % |
| | II.15 | $O-\overset{O}{\overset{\|}{C}}-CH{=}CH-CH_3$ | =O | H | H | $(H_3C-CH{=}CH-\overset{O}{\overset{\|}{C}})_2O$, Pyr. DMAP | 18 h/RT | a) | 1 | 62 % |
| | II.16 | 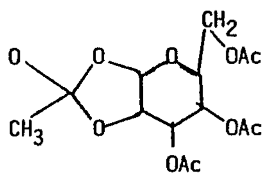 | =O | H | H | α-Bromtetraacetylglukose, Collidin, $AgClO_4$, Molsieb 3Å | 3 h, -10°C | d) | 6 | 38 % |
| 10 | II.17 | OH | =O | H | 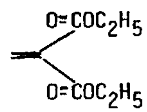 | Mesoxalsäurediethylester, $NEt_3$ | 2 h, -20°C 4 h/RT | 1) | 10 | 84 % |
| | II.18 | O-C(O)-Cyclohexyl | =O | H | H | Cyclohexancarbonsäureanhydrid, DMAP, Pyr., $CH_2Cl_2$ | 12 h/RT | a) | 1 | 61 % |
| 3 | II.19 | =O | =O | H | H | PCC, $CH_2Cl_2$ | 3 h/RT | e) | 3 | 54 % |
| | II.20 | $O-\overset{O}{\overset{\|}{C}}-NH-benzyl$ | =O | H | H | Benzylisocyanat, $NEt_3$ Ether | 12 h/RT | b) | 2 | 43 % |

Fortsetzung Tabelle 1

| Beisp.-Nr. | Verbindung | $R^1$ | $R^2$ | $A^1$ | $A^2$ | Reagenz | Bedingungen | Verfahren | Ausf.gem. Bsp. Nr. | Ausbeute |
|---|---|---|---|---|---|---|---|---|---|---|
| | II.21 | 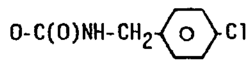 | =O | H | H | 4-Chlorbenzylisocyanat, $NEt_3$ | 18 h/RT | b) | 2 | 47 % |
| | II 22 | $O-C(O)-CH_2-O-C_6H_4-4C_7H_{15}$ | =O | H | H | 4-Heptylphenoxyessigsäure-anhydrid, Pyr., DMAP | 18 h/RT | a) | 1 | 67 % |
| | II.23 | 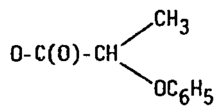 | =O | H | H | 2-Phenoxypropionsäure-anhydrid, DMAP, Pyr. | 24 h/RT | a) | 1 | 48 % |
| | II.24 | O-C(O)-(2,4-Dimethoxy-phenyl) | =O | H | H | 2,4-Dimethoxybenzoesäure, Pyr., DMAP | 18 h/RT | a) | 1 | 70 % |
| | II.25 | $O-C(O)-CH_2-O-C_6H_5$ | =O | H | H | Phenoxyessigsäureanhydrid DMAP, Pyr. | 24 h/RT | a) | 1 | 74 % |
| | II.26 | $O-C(O)-(CH_2)_8-CH_3$ | =O | H | H | Decansäureanhydrid, DMAP, Pyr. | 18 h/RT | a) | 1 | 70 % |
| | II.27 | 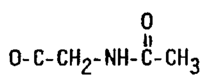 | =O | H | H | Acetylglycin, DCC, DMAP | 36 h/RT | a) | 8 | 51 % |
| | II.28 | 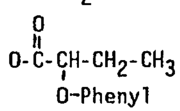 | =O | H | H | 2-Phenoxybuttersäureanhydrid, DMAP, Pyr. | 18 h/PT | a) | 1 | 53 % |
| 4 | II'.1 | OH | $R^5=C_6H_{13}$ | H | H | Hexylamin, THF | 72 h/RT | c) | 4 | 94 % |

Fortsetzung Tabelle 1

| Beisp.-Nr. | Verbindung | $R^1$ | $R^2$ | $A^1$ | $A^2$ | Reagenz | Bedingungen | Verfahren | Ausf.gem. Bsp. Nr. | Ausbeute |
|---|---|---|---|---|---|---|---|---|---|---|
| | II.29 | $O-CH_2-OC_2H_4-OCH_3$ | =O | H | H | MEM-chlorid, Ethyldiiso-propylamin | 12 h/RT | b) | 9 | 70 % |
| | II.30 | $O-\overset{O}{\overset{\parallel}{C}}-NH-(2,4-F_2-C_6H_3)$ | =O | H | H | $(2,4-F_2C_6H_3)-N=C=O$, | 22 h/RT | b) | 2 | 48 % |
| 5 | II.31 | $O-\underset{O}{\underset{\parallel}{C}}-CH_2-\underset{O}{\underset{\parallel}{C}}-CH_3$ | =O | H | H | Diketen | 24 h/RT | k) | 5 | 32 % |
| | II.32 | $OC_2H_5$ | =O | $C_2H_5$ | $C_2H_5$ | $C_2H_5-I$, $Ag_2O$, DMF | 6 h/RT | h) | 7 | 65 % |
| | II'.2 | OH $R^5$=$-(CH_2)_2-C_6H_4-4-OCH_3$ | | H | H | $NH_2-(CH_2)_2-C_6H_4-4-OCH_3$, THF | 2 h/RT | c) | 4 | 84 % |
| 13 | I.1 | - | =O | H | H | 1) Tosylchlorid, Pyr.<br>2) DBU, Toluol | 12 h/RT<br>3 h/80°C | b) | 13 | 48 % |
| | II.33 | H | =O | H | H | $H_2$, Pd(C), MeOH | RT | g) | 14 | 92 % |
| | II.34 | OH | =O | $CH_2C_6H_5$ | $CH_2C_6H_5$ | Benzylbromid, $Ag_2O$, DMF | 24 h/RT | h) | 7 | 73 % |
| | II.35 | $O-C(O)-C_6H_4F$ | =O | H | H | 4-F-Benzoesäure, DCC, DMAP | 12 h/RT | a) | 8 | 70 % |
| | II.36 | $O-\underset{O}{\underset{\parallel}{C}}-(CH_2)_5NHC(O)CH_3$ | =O | H | H | N-Acetyl-hexancarbonsäure, DCC, DMAP | 12 h/RT | a) | 8 | 46 |
| | II.37 | 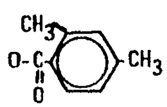 | =O | H | H | $2,4(CH_3)_2$-Benzoesäure DCC, DMAP | 24 h/RT | a) | 8 | 66 % |
| 8 | II.38 | 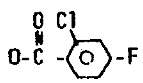 | =O | H | H | 4-F-2-Cl-Benzoesäure, DCC DMAP | 12 h/RT | a) | 8 | 60 % |

Fortsetzung Tabelle 1

| Beisp.-Nr. | Verbindung | $R^1$ | $R^2$ | $A^1$ | $A^2$ | Reagenz | Bedingungen | Verfahren | Ausf.gem. Bsp. Nr. | Ausbeute |
|---|---|---|---|---|---|---|---|---|---|---|
| | II.39 | O-C(O) 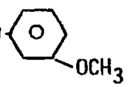 | =O | H | H | 3-$OCH_3$-Benzoesäureanhydrid DMAP, Pyr., $CHCl_3$ | 24 h/RT | a) | 1 | 71 % |
| 7 | II.40 | $OCH_3$ | =O | $CH_3$ | $CH_3$ | $CH_3I$, $Ag_2O$, DMF | 6 h/RT | k) | 7 | 92 % |
| | II.41 | $OCH_3$ | -OH | $CH_3$ | $CH_3$ | $NaBH_4$, $CH_3OH$ | 4 h/RT | f) | aus Verb. II.40 nach Bsp. 13 | 37 % |
| 6 | II.42 | 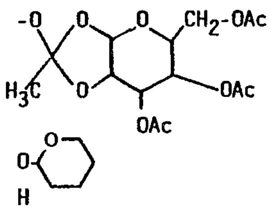 | =O | H | H | α-Brom-tetraacetylgalaktose | 24 h/RT | d) | | 47 % |
| 12 | II.43 | | =O | H | H | Dihydropyran, PPTS | 24 h/RT | b) | 12 | 74 % |
| 14 | II.44 | H | =O | H | H | Pd(C), THF, $H_2$ | 1 h/RT | g) | 14 | 92 % |
| | II.45 | OH | -N(H)-hexyl | H | H | Pt(C), THF, $H_2$ | 3 h/RT | g) | aus Verb. II'.1 nach Bsp. 14 | 80 % |
| | II'.3 | OH | $R^5=C_4H_9$ | H | H | n-Butylamin, THF Molsieb 4Å | 24 h/RT | c) | 4 | 60 % |
| | II'.4 | OH | $R^5=-(CH_2)_2-C_6H_5$ | H | H | Phenylethylamin, THF | 2 h/RT | c) | 4 | 81 % |

Pyr. = Pyridin
DMAP = N,N-Dimethylaminopyridin
TMSCl = Trimethylsilylchlorid
PCC = Pyridiniumchlorochromat
DCC = Dicyclohexylcarbodiimid
DBU = 1,8-Diazobicyclo[5.4.0]undec-7-en
$NEt_3$ = Triethylamin
THF = Tetrahydrofuran
DMF = Dimethylformamid
PPTS = Pyridinium-p-toluolsulfonat

## Hemmung der Cholesterin Biosynthese

Die Hemmung der Cholesterin Biosynthese durch die erfindungsgemäßen Verbindungen wurde in Zellkulturversuchen mit HEP-G2-Zellen über die Wirkung auf den Einbau von $^{14}$C-Natriumacetat in Chole-

sterin nachgewiesen. Dazu wurden Monolayers von HEP-G2-Zellen in lipoproteinfreiem Nährmedium mit entsprechenden Konzentrationen der erfindungsgemäßen Verbindungen eine Stunde vorinkubiert; nach Zugabe des $^{14}C$ markierten Präkursors $^{14}C$-Natriumacetat wurde die Inkubation für 3 Stunden fortgesetzt. Danach wurde ein Teil der Zellen alkalisch verseift, nach vorheriger Zugabe eines internen Standards von $^{3}H$-Cholesterin. Die Lipide der verseiften Zellen wurden mit Chloroform-Methanol extrahiert. Dieses Lipidgemisch wurde nach Zusatz von Trägercholesterin präparativ dünnschichtchromatographisch getrennt, die Cholesterinbande nach dem Sichtbarmachen isoliert und die aus dem $^{14}C$-Präkursor gebildete Menge $^{14}C$-Cholesterin szintigraphisch bestimmt. In einem aliquoten Teil der Zellen wurde Zellprotein bestimmt, so daß die in der Zeiteinheit pro mg Zellprotein aus $^{14}C$-Präkursor gebildete Menge an $^{14}C$-Cholesterin berechnet werden konnte. Zum Vergleich für die Hemmwirkung einer zugesetzten erfindungsgemäßen Verbindung diente die Kontrolle, so daß direkt die Hemmung der Cholesterinbiosynthese bei einer bestimmten molaren Konzentration der erfindungsgemäßen Verbindung im Medium angegeben werden konnte. Zur Beurteilung der Wirkungstärke der erfindungsgemäßen Verbindungen wurde die Hemmwirkung von Lovastatin im selben Versuchsansatz bestimmt. In aliquoten Anteilen der Zellkultur wurde die Intaktheit der Zellkultur und die fehlende Zellschädigung durch Einwirkung der erfindungsgemäßen Verbindung morphologisch (Lichtmikroskop) beurteilt und biochemisch durch Bestimmung des Release von Laktat-Dehydrogenase ins Inkubationsmedium gemessen.

Das Ergebnis dieser Untersuchung ist in Tabelle 2 wiedergegeben. Die Wirkung der erfindungsgemäßen Substanzen als Cholesterin-Biosynthese-Hemmer ist deutlich zu erkennen. In den geprüften Konzentrationen hatten die erfindungsgemäßen Verbindungen im Zeitraum von 4 Stunden keine cytotoxische Wirkung.

**Tabelle 2**

Hemmung der Cholesterin-Biosynthese in HEP-G2-Zellkultur

| Substanz | Konzentration im Medium [M] | % Cholesterin-Biosynthese-Hemmung | $IC_{50}$ der Hemmung | Vergleichsreihe für Lovastatin im selben Versuchsansatz $IC_{50}$ [M] | % Hemmung |
|---|---|---|---|---|---|
| II.44 | $5\cdot10^{-8}$ | 36 | $3\cdot10^{-8}$ | 6.10-8 | 41 |
| II.35 | $10^{-9}$ | 46 | $6\cdot10^{-9}$ | $6\cdot10^{-9}$ | |
| II.36 | $10^{-9}$ | 55 | | $6\cdot10^{-9}$ | |
| II.3 | $5\cdot10^{-10}$ | 0 | | $6\cdot10^{-8}$ | |
| | $5\cdot10^{-9}$ | 9 | | $6\cdot10^{-8}$ | |
| | $10^{-8}$ | 39 | | $2,2\cdot10^{-8}$ | |
| | $5\cdot10^{-8}$ | 49 | | $2,2\cdot10^{-8}$ | |
| | $10^{-7}$ | 81 | | $1,3\cdot10^{-8}$ | |
| II.27 | $10^{-8}$ | 68 | | $6,5\cdot10^{-8}$ | |
| | $10^{-7}$ | 39 | | $2,3\cdot10^{-8}$ | |
| I.1 | $5\cdot10^{-8}$ | 43 | | $6,5\cdot10^{-8}$ | |
| II.30 | $5\cdot10^{-8}$ | 60 | | $6,5\cdot10^{-8}$ | |
| II'.4 | $10^{-7}$ | 73 | | $2,3\cdot10^{-8}$ | |
| II.22 | $10^{-7}$ | 44 | | $2,2\cdot10^{-8}$ | |
| II.25 | $10^{-7}$ | 52 | | $2,2\cdot10^{-8}$ | |

**Fortsetzung Tabelle 2**

| Substanz | Konzentration im Medium [M] | % Cholesterin-Biosynthese-Hemmung | $IC_{50}$ der Hemmung | Vergleichsreihe für Lovastatin im selben Versuchsansatz $IC_{50}$ [M] | % Hemmung |
|---|---|---|---|---|---|
| II.8 | $2,5\cdot10^{-9}$ | 51 | | $10^{-8}$ | |
| | $10^{-7}$ | 69 | | | |
| II.11 | $2,5\cdot10^{-9}$ | 57 | | $10^{-8}$ | |
| | $10^{-7}$ | 73 | | | |
| II.10 | $2,5\cdot10^{-9}$ | 48 | | $10^{-8}$ | |
| | $10^{-7}$ | 59 | | | |
| II.16 | $10^{-9}$ | 47 | | $10^{-8}$ | |
| II.13 | $10^{-9}$ | 37 | | $10^{-8}$ | |
| | $10^{-8}$ | 44 | | | |
| II.4 | $10^{-7}$ | 68 | | $10^{-8}$ | |
| II.1 | $10^{-7}$ | 13 | | $10^{-8}$ | |

Die Hemmung der hepatischen Cholesterinbiosynthese hat Auswirkungen auf die Verminderung von Serum-Lipiden, wie im chronischen Versuch an der männlichen Ratte nachgewiesen werden konnte (Tabelle 2).

**Versuchsdurchführung**

Gruppen männlicher Ratten des Stammes HOE: WISKf (SPF 71) mit einem Ausgangsgewicht von etwa 240 g erhielten täglich morgens die erfindungsgemäßen Verbindungen in Polyethylenglykol 400 per

Schlundsonde, wobei die jeweilige Kontrollgruppe nur das Vehikel erhielt. 24 Stunden nach der letzten (7.) Applikation und nach 24 Stunden Futterentzug wurde Blut entnommen und im gewonnen Serum die Lipoproteine aus dem Pool einer Rattengruppe mittels der präparativen Ultrazentrifugentechnik getrennt. Hierbei wurden folgende Dichtegrenzen für die Trennung von VLDL, LDL und HDL benutzt:

| | |
|---|---|
| VLDL | 1,006 |
| LDL | 1,04 |
| HDL | 1,21 |

VLDL = very low density lipoprotein
LDL = low density lipoprotein
HDL = high density lipoprotein

Für die Bestimmung des Cholesterins und der Triglyceride wurden vollenzymatische Methoden nach Boehringer/Mannheim benutzt, für die Bestimmung des Proteins die Methode von Lowry et al. J. Biol. Chem., 193, 265 (1951) herangezogen. Wie anhand des Beispiels II.3 (s. Tabelle 3) dargelegt ist, führte die 7-tägige orale Applikation in einer um ein Drittel geringeren Dosis als das Standard Präparat Clofibrat zu einer deutlichen Verminderung von LDL und VLDL, bei Erhöhung der HDL-Fraktion. Daraus resultiert eine Erhöhung des vasoprotektiven Index (HDL-Cholesterin : LDL-Cholesterin) mit einer ca. 8-fach höheren Potency als Clofibrat. Die Anwendung der Substanz wurde von den Ratten anstandslos vertragen. Im Gegensatz zu Clofibrat erfolgte keine Hepatomegalie.

**Tabelle 3**

| Substanz | Dosis mg/kg/Tag | Beeinflussung der Serumlipoproteine im Vergleich zur Kontrollgruppe in % VLDL-C | LDL-C | HDL-C | VLDL-Protein | LDL-Protein | HDL-Protein | Vasoprotektiv Index HDL-C LDL-C | im Vergleich zur Kontrolle | VLDL Trigly-cerid |
|---|---|---|---|---|---|---|---|---|---|---|
| II.3 | 30 | -21 | -19 | +22 | - 8 | -14 | + 9 | 4,88 | + 51 % | - 3 |
| Clofibrat | 100 | -56 | -47 | -36 | - 4 | -13 | -14 | 3,87 | + 19 % | - 2 |
| Kontrolle | | | | | | | | 3,24 | =100 % | |

VLDL-C = VLDL-Cholesterin
LDL-C = LDL-Cholesterin
HDL-C = HDL-Cholesterin

## Ansprüche

**1.** 10-Ring-Lactone der Formeln I und II

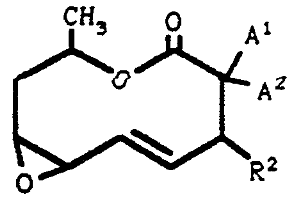

(I)

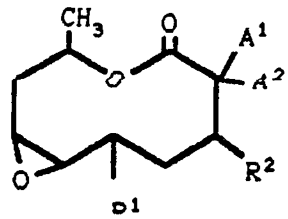

(II)

in denen
$A^1$ und $A^2$
gleich oder verschieden sind und H oder $C_1$-$C_4$-Alkyl bedeuten, wobei der $C_1$-$C_4$-Alkylrest unsubstituiert ist oder ein- oder mehrfach substituiert ist mit OH oder Phenyl, wobei der Phenylrest seinerseits unsubstituiert ist oder ein- oder mehrfach substituiert ist mit Halogen, $CF_3$ oder $C_1$-$C_3$-Alkyl
oder worin
$A^1$ und $A^2$
zusammen einen Rest der Formel

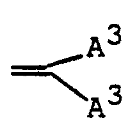

bilden, worin
$A^3$ und $A^{3'}$ gleich oder verschieden sind und
$C_1$-$C_2$-Alkoxycarbonyl, Phenyl oder $C_1$-$C_4$-Alkyl bedeuten
oder worin
$A^1$ und $A^2$
zusammen einen Rest der Formel

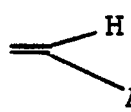 oder 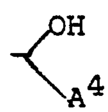

datstelen, worin $A^4$ Phenyl oder $C_1$-$C_6$-Alkyl bedeutet,

$R^1$ -H, =O oder -$OR^3$ bedeutet und
$R^2$ =O, -$OR^4$, =N-$R^5$ oder 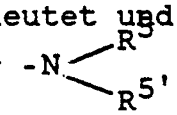

bedeutet, wobei im Falle
der Formel II für $R^2$ = =N-$R^5$ und $A^1$ = H diese auch als Verbindung der Formel II'

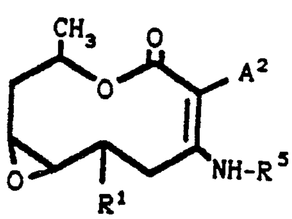

(II')

vorliegen kann
wobei

$R^3$
H, $C_1$-$C_8$-Alkyl, Benzyl, Allyl, MEM, MOM, SEM, THP, 4'-Methoxytetrahydropyran-4'-yl, Trimethylsilyl, Triethylsilyl, Dimethylphenylsilyl, Dimethylcyclohexylsilyl, Dimethyltertiärbutylsilyl oder einen Sulfonsäureester der Formel $-SO_2R^7$ bedeutet, wobei $R^7$ $C_1$-$C_{10}$-Alkyl, Phenyl oder p-Methylphenyl bedeutet
oder
$R^3$
ein Mono- oder Disaccharid darstellt, dessen OH-Gruppen ungeschützt sind oder mit Schutzgruppen geschützt sind
oder
$R^3$
einen Rest der Formel

$$-\overset{\overset{X}{\|}}{C}-Z-R^8 \quad \text{oder} \quad -\overset{\overset{X}{\|}}{C}-Z-(CH_2)_n-R^8$$

darstellt, wobei
X O oder S

$$Z \quad O, \; -\overset{|}{N}-R^8, \; -\overset{|}{N}-(CH_2)_n-R^8 \; \text{oder} \; -\overset{|}{N}H$$

n 1 bis 3 bedeutet und
$R^8$ $C_1$-$C_8$-Alkyl, $C_3$-$C_9$-Cycloalkyl, Aryl, Pyridyl, Pyrimidyl oder Pyrazinyl bedeutet
oder
$R^3$
einen Rest der Formel

$$-\overset{\overset{O}{\|}}{C}-R^9$$

darstellt,
wobei
$R^9$
$C_1$-$C_{20}$-Alkyl, $C_2$-$C_{16}$-Alkenyl mit 1-3 Doppelbindungen,
$C_2$-$C_{16}$-Alkinyl mit 1-3- Dreifachbindungen,
$C_3$-$C_9$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Aryl, Aryloxy, Heteroaryl, Heteroaryloxy, $NHCOCH_3$, $COA^5$, $SO_3A^5$, wobei $A^5$ $C_1$-$C_4$-Alkyl oder H bedeutet
und wobei
alle genannten Aryl-, Heteroaryl-, Pyridyl-, Pyrimidyl- und Pyrazinylreste unsubstituiert sind oder ein- oder mehrfach substituiert sind mit
Halogen, Nitro, Cyano, Hydroxy, Trifluormethyl, Amino, Carboxy, $C_1$-$C_4$-Alkyl-carbonyl, $C_1$-$C_4$-Alkoxycarbonyl, $C_1$-$C_{10}$-Alkyl, $C_1$-$C_4$-Alkoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$, sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H.

2. 10-Ring-Lactone der Formeln I und II nach Anspruch 1, in denen
$A^1$ und $A^2$
gleich oder verschieden sind und H oder $C_1$-$C_2$-Alkyl bedeuten, wobei der $C_1$-$C_2$-Alkylrest unsubstituiert ist oder einfach substituiert ist mit Phenyl, wobei der Phenylrest seinerseits unsubstituiert ist oder einfach substituiert ist mit Halogen, $CF_3$ oder $C_1$-$C_3$-Alkyl
oder worin
$A^1$ und $A^2$
zusammen einen Rest der Formel

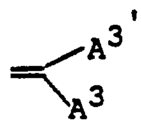

bilden, worin
$A^3$ und $A^{3'}$ gleich oder verschieden sind und $C_1$-$C_2$-Alkoxycarbonyl oder Phenyl bedeuten
oder worin
$A^1$ und $A^2$
zusammen einen Rest der Formel

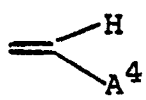

darstellen,
worin $A^4$ Phenyl bedeutet,

$R^1$ -H, =O oder $-OR^3$ bedeutet und
$R^2$ =O, $-OR^4$, $=N-R^5$ oder 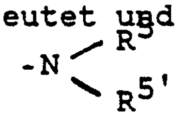

bedeutet, wobei im Falle der Formel II für $R^2$ = $=N-R^5$ und $A^1$ = H diese auch als Verbindung der Formel II', wie in Anspruch 1 angegeben, vorliegen kann
wobei
$R^3$
H, $C_1$-$C_8$-Alkyl, Benzyl, Allyl, MEM, MOM, SEM, THP, 4'-Methoxytetrahydropyran-4'-yl, Trimethylsilyl, Triethylsilyl, Dimethylphenylsilyl, Dimethylcyclohexylsilyl, Dimethyltertiärbutylsilyl oder einen Sulfonsäureester der Formel $-SO_2R^7$ bedeutet, wobei $R^7$ Methyl, Phenyl oder p-Methylphenyl bedeutet
oder
$R^3$
einen Pyranosylrest darstellt, dessen OH-Gruppen ungeschützt sind oder mit Schutzgruppen geschützt sind
oder
$R^3$
einen Rest der Formel

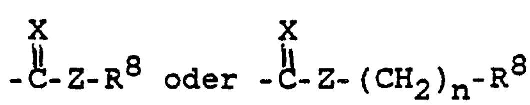

darstellt, wobei

X O

Z O, $-N-R^8$, $-N-(CH_2)_n-R^8$ oder $-NH$

n 1 bis 3 bedeutet und

$R^8$ $C_1$-$C_8$-Alkyl, $C_3$-$C_6$-Cycloalkyl oder Aryl bedeutet

oder

$R^3$

einen Rest der Formel

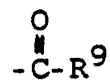

darstellt,

wobei

$R^9$

$C_1$-$C_{15}$-Alkyl, $C_2$-$C_{16}$-Alkenyl, $C_3$-$C_6$-Alkinyl, $C_3$-$C_6$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Phenyl, Phenyloxy, $NHCOCH_3$, $COA^5$, $SO_3A^5$, wobei $A^5$ $C_1$-$C_4$-Alkyl oder H bedeutet

und wobei

alle genannten Aryl- und Heteroarylreste unsubstituiert sind oder ein- oder mehrfach substituiert sind mit

Halogen, Cyano, Trifluormethyl, Carboxy, $C_1$-$C_4$-Alkyl-carbonyl, $C_1$-$C_4$-Alkoxy-carbonyl, $C_1$-$C_7$-Alkyl, $C_1$-$C_4$-Alkoxy, $NHCOCH_3$

und worin

$R^4$

unabhängig von $R^3$ die oben für $R^3$ angegebenen Bedeutungen hat und

$R^5$ und $R^{5'}$

gleich oder verschieden sind und Wasserstoff, $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$, sowie die physiologisch verträglichen Salze, ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H.

**3.** 10-Ring-Lactone der Formel I und II nach Anspruch 1 oder 2, in denen

$A^1$ und $A^2$

gleich oder verschieden sind und H oder Methyl bedeuten, wobei der Methylrest unsubstituiert ist oder einfach substituiert ist mit Phenyl,

oder worin

$A^1$ und $A^2$

zusammen einen Rest der Formel

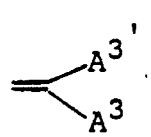

bilden, worin $A^3$ und $A^{3'}$ gleich sind und Äthoxycarbonyl bedeuten

$R^1$ -H, =O oder -$OR^3$ bedeutet und
$R^2$ =O, -$OR^4$, =N-$R^5$ oder 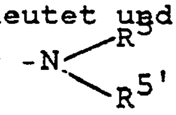

bedeutet, wobei im Falle der Formel II für $R^2$ = =N-$R^5$ und $A^1$ = H diese auch als Verbindung der Formel II', wie in Anspruch 1 angegeben, vorliegen kan
wobei
$R^3$
H, Methyl, Benzyl, THP, Trimethylsilyl, Dimethyltertiärbutylsilyl oder einen Sulfonsäureester der Formel -$SO_2R^7$ bedeutet, wobei
$R^7$ p-Methylphenyl bedeutet
oder
$R^3$
einen Glucosyl-, Galactosyl- oder Lactosylrest darstellt, dessen OH-Gruppen ungeschützt sind oder mit Acetylgruppen oder Benzylgruppen geschützt sind, wobei diese genannten Zuckerreste entweder glykosidisch gebunden sind oder über einen Orthoester verknüpft sind
oder
$R^3$
einen Rest der Formel

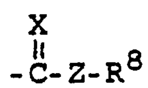 oder 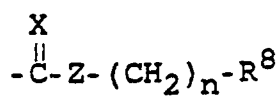

darstellt, wobei
X O

Z -NH

n 1 bedeutet und
$R^8$ $C_4$-$C_8$-Alkyl oder Aryl bedeutet
oder
$R^3$
einen Rest der Formel

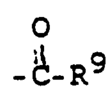

darstellt,
wobei
$R^9$
$C_1$-$C_{15}$-Alkyl, $C_2$-$C_8$-Alkenyl, $C_3$-$C_6$-Cycloalkyl, Phenyl oder Thienyl oder Furyl bedeutet, wobei die Alkyl-, Alkenyl-, und Cycloalkylreste unsubstituiert sind oder ein- oder mehrfach substituiert sind mit $COOA^5$, F, Cl, Br,Phenyl, Phenyloxy, $NHCOCH_3$, $COA^5$, wobei $A^5$ Methyl oder H bedeutet und wobei alle genannten Aryl- oder Phenylreste unsubstituiert sind oder ein- oder mehrfach substituiert sind mit F, Cl, Br, Trifluormethyl, Carboxy, $C_1$-$C_4$-Alkyl-carbonyl, $C_1$-$C_4$-Alkoxy-carbonyl, $C_1$-$C_7$-Alkyl, Methoxy, $NHCOCH_3$
und worin
$R^4$
unabhängig von $R^3$ die oben für $R^3$ angegebenen Bedeutungen hat und
$R^5$ und $R^{5'}$

gleich oder verschieden sind und Wasserstoff, $C_1$-$C_6$-Alkyl, Benzyl, Phenyl oder Phenylethyl bedeuten, wobei die genannten Reste unsubstituiert sind oder substituiert sind mit F, Cl, Br oder $OCH_3$,
sowie die physiologisch verträglichen Salze,
ausgenommen die Verbindung der Formel I, in der $R^2$ -$OR^4$ bedeutet mit $R^4$ = H sowie ebenfalls ausgenommen die Verbindung der Formel II, in der $R^1$ -$OR^3$ und $R^2$ =O bedeutet mit $R^3$ = H.

**4.** Verfahren zur Herstellung von 10-Ring-Lactonen gemäß Formel I und II, nach Anspruch 1, dadurch gekennzeichnet, daß man

a) die Verbindung der Formel III

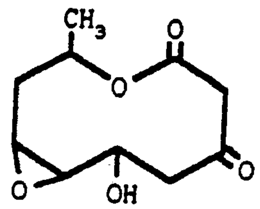

(III)

umsetzt mit einer Verbindung der Formel IV

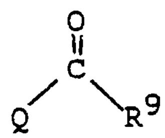

(IV)

worin

Q

OH, Chlorid, Bromid, Imidazolid oder ein Säureanhydrid bedeutet und

$R^9$

die in Anspruch 1 zu Formel I und II angegebene Bedeutungen hat,

oder daß man

b) die Verbindung der Formel III umsetzt mit einer Verbindung ausgewählt aus:
Dihydropyran, 4-Methoxy-5,6-dihydropyran, $C_1$-$C_8$-Alkyl-, Benzly-, Allylchlorid, -bromid, -jodid oder MEM-, MOM-, SEM-chlorid, Trimethylsilyl-, Triethylsilyl-, Dimethylphenylsilyl-, Dimethylcyclohexylsilyl-, Dimethyltertiärbutylsilylchlorid, Sulfonsäurehalogeniden der Formel Hal-$SO_2R^7$, wobei Hal Chlor, Brom oder Jod bedeutet und $R^7$ die oben angegebenen Bedeutungen hat, Isocyanaten der Formel
O = C = N-$R^8$ oder
O = C = N-$(CH_2)_n$-$R^8$, Isothiocyanaten der Formel
S = C = N-$R^8$ oder
S = C = N-$(CH_2)n$-$R^8$, Carbaminsäurehalogeniden der Formel
Hal'-CO-N-$(R^8)_2$ oder
Hal'-CO-N-$[(CH_2)_n$-$R^8]_2$, Thiocarbaminsäurehalogeniden der Formel Hal'-CS-N-$(R^8)_2$ oder
Hal'-CS-N-$[(CH_2)_n$-$R^8]_2$' oder Verbindungen der Formeln Hal'-C(O)-O-$R^8$, Hal'-CO-$(CH_2)_n$-$R^8$, Hal'-CS-O-$R^8$ oder
Hal'-CS-O-$(CH_2)_n$-$R^8$
wobei n und $R^8$ die in Anspruch 1 in Formel I und II angegebenen Bedeutungen haben und Hal' Chlor oder Brom bedeutet,

oder daß man

c) die Verbindung der Formel III umsetzt mit einer Verbindung der Formel V

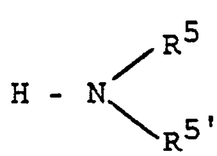

(V)

worin $R^5$ und $R^{5'}$ wie in Anspruch 1 in Formel I und II angegeben definiert sind, und die entstehende C-C-Doppelbindung in der Verbindung der Formel II' gegebenenfalls hydriert,

oder daß man

d) die Verbindung der Formel III umsetzt mit einer Verbindung der Formel VI

$R^3$ - Hal (VI)

wobei $R^3$ ein Mono- oder Disaccharid darstellt und Hal wie oben definiert ist,

oder daß man

e) die Verbindung der Formel III zu einer Verbindung der Formel VII oxidiert

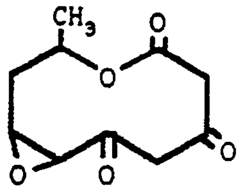

(VII)

oder daß man

f) die Verbindung der Formel III zu einer Verbindung der Formel

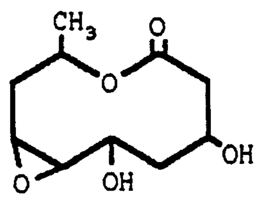

reduziert,

oder daß man

g) eine Verbindung der Formel III zum Enon

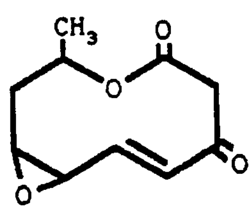

eliminiert und gegebenenfalls die C-C-Doppelbindung hydriert, wobei eine Verbindung der Formel

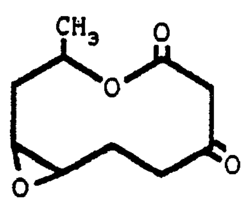

entsteht,

oder daß man

h) die Verbindung der Formel III umsetzt mit einer Verbindung der Formel VIII und/oder VIII'

$A^1$ - Abg (VIII)
$A^2$ - Abg (VIII')

worin $A^1$ und $A^2$ bis auf die Bedeutung

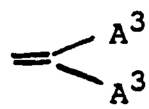

wie in Anspruch 1 in Formel I und II definiert sind und Abg, Chlor, Brom, Jod, Sulfat, Mesylat oder Tosylat bedeutet

oder daß man

i) die Verbindung der Formel III umsetzt mit einer Verbindung der Formel IX

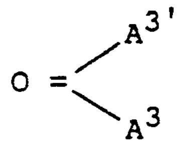

(IX)

worin $A^3$ und $A^{3'}$ wie in Anspruch 1 in Formel I und II definiert ist,

oder daß man

j) die Verbindung der Formel III umsetzt mit einer Verbindung der Formel X

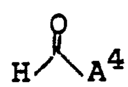

(X)

worin $A^4$ wie in Anspruch 1 in Formel I und II definiert ist

oder daß man

k) die Verbindung der Formel III umsetzt mit Diketen

wobei eine Verbindung der Formel II entsteht, mit $R^1$ = O-C(O)-$CH_2$-C(O)-$CH_3$

oder daß man

l) eine Verbindung der Formel III mit einem Mesoxalsäurediester umsetzt, wobei eine Verbindung der Formel II entsteht mit

$A^1$ und $A^2$ = 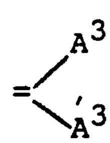

wobei $A^3$ und $A^{3'}$ wie in Anspruch 1 in Formel I und II definiert

oder daß man

zwei oder mehrere der vorstehend genannten Verfahrensvarianten a-l hintereinander ausführt und daß man anschließend gegebenenfalls die Verbindungen I oder II in ihre physiologisch verträglichen Salze überführt.

5. 10-Ring-Lactone der Formeln I und II nach einem der Ansprüche 1 bis 3, jedoch einschließlich der Verbindung der Formel I in der $R^2$ -$OR^4$ bedeutet mit $R^4$ = H, sowie einschließlich der Verbindung der Formel II, in der $R^1$ -$OR^3$ und $R^2$ =O bedeutet, mit $R^3$ = H, zur Herstellung eines Arzneimittels mit Cholesterin-Biosynthese hemmender Wirkung.

6. Verbindungen der Formel I und II nach einem der Ansprüche 1 bis 3 zur Anwendung als Arzneimittel.

7. Verbindungen der Formeln I und II nach einem der Ansprüche 1 bis 3 zur Anwendung als Arzneimittel mit lipidsenkender Wirkung.

8. Arzneimittel, enthaltend mindestens eine Verbindung der Formel I und/oder II nach einem der Ansprüche 1 bis 3.

9. Verfahren zur Herstellung eines Arzneimittels, dadurch gekennzeichnet, daß man in das Arzneimittel mindestens eine Verbindung der Formel I und/oder II nach einem der Ansprüche 1 bis 3 einverbleibt.

Patentansprüche für folgende Vertragsstaaten: ES, GR

1. Verfahren zur Herstellung der 10-Ring-Lactone der Formeln I und II

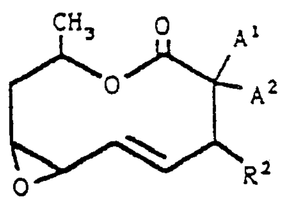
(I)
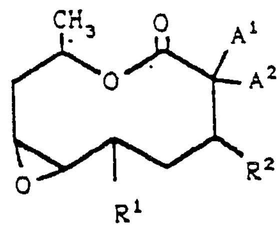
(II)

in denen
$A^1$ und $A^2$
gleich oder verschieden sind und H oder $C_1$-$C_4$-Alkyl bedeuten, wobei der $C_1$-$C_4$-Alkylrest unsubstituiert ist oder ein- oder mehrfach substituiert ist mit OH oder Phenyl, wobei der Phenylrest seinerseits unsubstituiert ist oder ein- oder mehrfach substituiert ist mit Halogen, $CF_3$ oder $C_1$-$C_3$-Alkyl
oder worin
$A^1$ und $A^2$
zusammen einen Rest der Formel

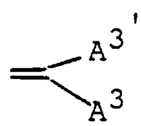

bilden, worin $A^3$ und $A^{3'}$ gleich oder verschieden sind und $C_1$-$C_2$-Alkoxycarbonyl, Phenyl oder $C_1$-$C_4$-Alkyl bedeuten
oder worin
$A^1$ und $A^2$
zusammen einen Rest der Formel darstellen, worin $A^4$ Phenyl oder

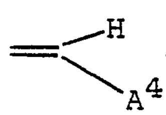 oder 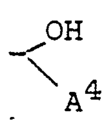

$C_1$-$C_6$-Alkyl bedeutet,

$R^1$ -H, =O oder -$OR^3$ bedeutet und
$R^2$ =O, -$OR^4$, =N-$R^5$ oder 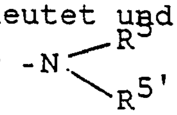

bedeutet, wobei im Falle der Formel II für $R^2$ = $=N-R^5$ und $A^1$ = H diese auch als Verbindung der Formel II'

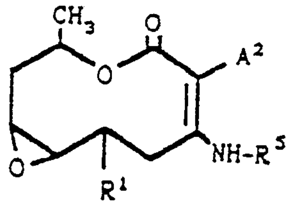

(II')

vorliegen kann
wobei
$R^3$
H, $C_1$-$C_8$-Alkyl, Benzyl, Allyl, MEM, MOM, SEM, THP, 4'-Methoxytetrahydropyran-4'-yl, Trimethylsilyl, Triethylsilyl, Dimethylphenylsilyl, Dimethylcyclohexylsilyl, Dimethyltertiärbutylsilyl oder einen Sulfonsäureester der Formel $-SO_2R^7$ bedeutet, wobei $R^7$ $C_1$-$C_{10}$-Alkyl, Phenyl oder p-Methylphenyl bedeutet
oder
$R^3$
ein Mono- oder Disaccharid darstellt, dessen OH-Gruppen ungeschützt sind oder mit Schutzgruppen geschützt sind
oder
$R^3$
einen Rest der Formel

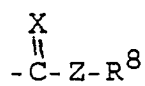 oder 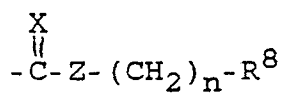

darstellt, wobei
X O oder S

Z O, $-N-R^8$, $-N-(CH_2)_n-R^8$ oder $-NH$

n 1 bis 3 bedeutet und
$R^8$ $C_1$-$C_8$-Alkyl, $C_3$-$C_9$-Cycloalkyl, Aryl, Pyridyl, Pyrimidyl oder Pyrazinyl bedeutet
oder
$R^3$
einen Rest der Formel

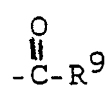

darstellt,
wobei
$R^9$
$C_1$-$C_{20}$-Alkyl, $C_2$-$C_{16}$-Alkenyl mit 1-3 Doppelbindungen, $C_2$-$C_{16}$-Alkinyl mit 1-3 Dreifachbindungen, $C_3$-$C_9$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Aryl, Aryloxy Heteroaryl, Heteroaryloxy, $NHCOCH_3$, $COA^5$, $SO_3A^5$, wobei $A^5$ $C_1$-$C_4$-Alkyl oder H bedeutet
und wobei
alle genannten Aryl-, Heteroaryl-, Pyridyl-, Pyrimidyl- und Pyrazinylreste unsubstituiert sind oder ein- oder mehrfach substituiert sind mit

Halogen, Nitro, Cyano, Hydroxy, Trifluormethyl, Amino, Carboxy, $C_1$-$C_4$-Alkyl-carbonyl, $C_1$-$C_4$-Alkoxy-carbonyl, $C_1$-$C_{10}$-Alkyl, $C_1$-$C_4$-Alkoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$, sowie die physiologisch verträglichen Salze,
ausgenommen die Verbindung der Formel I, in der $R^2$ -$OR^4$ bedeutet mit $R^4$ = H sowie ebenfalls ausgenommen die Verbindung der Formel II, in der $R^1$ -$OR^3$ und $R^2$ =O bedeutet mit $R^3$ = H, dadurch gekennzeichnet, daß man

a) die Verbindung der Formel III

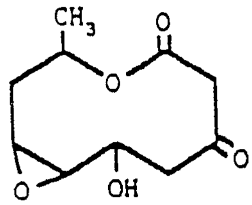

(III)

umsetzt mit einer Verbindung der Formel IV

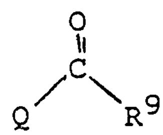

(IV)

worin
Q
OH, Chlorid, Bromid, Imidazolid oder ein Säureanhydrid bedeutet und
$R^9$
die in Anspruch 1 zu Formel I und II angegebene Bedeutungen hat,
oder daß man
b) die Verbindung der Formel III umsetzt mit einer Verbindung ausgewählt aus:
Dihydropyran, 4-Methoxy-5,6-dihydropyran, $C_1$-$C_8$-Alkyl-, Benzly-, Allylchlorid, -bromid, -jodid oder MEM-, MOM-, SEM-chlorid, Trimethylsilyl-, Triethylsilyl-, Dimethylphenylsilyl-, Dimethylcyclohexylsilyl-, Dimethyltertiärbutylsilylchlorid, Sulfonsäurehalogeniden der Formel Hal-$SO_2R^7$, wobei Hal Chlor, Brom oder Jod bedeutet und $R^7$ die oben angegebenen Bedeutungen hat, Isocyanaten der Formel
$O = C = N\text{-}R^8$ oder
$O = C = N\text{-}(CH_2)_n\text{-}R^8$, Isothiocyanaten der Formel
$S = C = N\text{-}R^8$ oder
$S = C = N\text{-}(CH_2)_n\text{-}R^8$, Carbaminsäurehalogeniden der Formel
$Hal'\text{-}CO\text{-}N\text{-}(R^8)_2$ oder
$Hal'\text{-}CO\text{-}N\text{-}[(CH_2)_n\text{-}R^8]_2$, Thiocarbaminsäurehalogeniden der Formel $Hal'\text{-}CS\text{-}N\text{-}(R^8)_2$ oder
$Hal'\text{-}CS\text{-}N[(CH_2)_n\text{-}R^8]_2$, oder Verbindungen der Formeln
$Hal'\text{-}C(O)\text{-}O\text{-}R^8$, $Hal'\text{-}CO\text{-}(CH_2)_n\text{-}R^8$, $Hal'\text{-}CS\text{-}O\text{-}R^8$ oder
$Hal'\text{-}CS\text{-}O\text{-}(CH_2)_n\text{-}R^8$
wobei n und $R^8$ die in Anspruch 1 in Formel I und II angegebenen Bedeutungen haben und Hal' Chlor oder Brom bedeutet,
oder daß man
c) die Verbindung der Formel III umsetzt mit einer Verbindung der Formel V

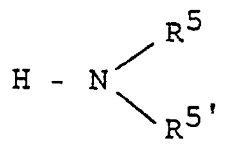

(V)

worin $R^5$ und $R^{5'}$ wie in Anspruch 1 in Formel I und II angegeben definiert sind, und die entstehende C-C-Doppelbindung in der Verbindung der Formel II' gegebenenfalls hydriert,

oder daß man

d) die Verbindung der Formel III umsetzt mit einer Verbindung der Formel VI

$R^3$ - Hal (VI)

wobei $R^3$ ein Mono- oder Disaccharid darstellt und Hal wie oben definiert ist,

oder daß man

e) die Verbindung der Formel III zu einer Verbindung der Formel VII oxidiert

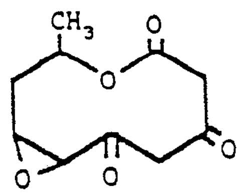

(VII)

oder daß man

f) die Verbindung der Formel III zu einer Verbindung der Formel

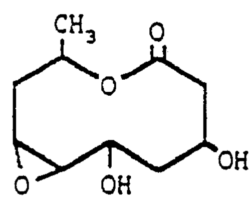

reduziert,

oder daß man

g) eine Verbindung der Formel III zum Enon

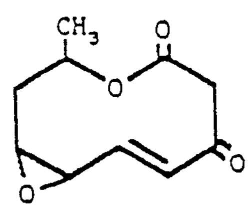

eliminiert und gegebenenfalls die C-C-Doppelbindung hydriert, wobei eine Verbindung der Formel

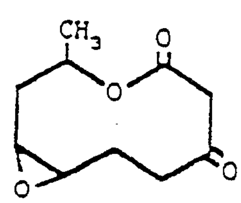

entsteht,

oder daß man

h) die Verbindung der Formel III umsetzt mit einer Verbindung der Formel VIII und/oder VIII'

$A^1$ - Abg (VIII)
$A^2$ - Abg (VIII')

worin $A^1$ und $A^2$ bis auf die Bedeutung

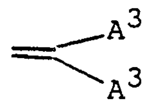

wie in Anspruch 1 in Formel I und II definiert sind und Abg, Chlor, Brom, Jod, Sulfat, Mesylat oder Tosylat bedeutet

oder daß man

i) die Verbindung der Formel III umsetzt mit einer Verbindung der Formel IX

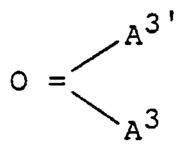

(IX)

worin $A^3$ und $A^{3'}$ wie in Anspruch 1 in Formel I und II definiert ist,

oder daß man

j) die Verbindung der Formel III umsetzt mit einer Verbindung der Formel X

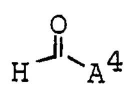

(X)

worin $A^4$ wie in Anspruch 1 in Formel I und II definiert ist

oder daß man

k) die Verbindung der Formel III umsetzt mit Diketen

wobei eine Verbindung der Formel II entsteht, mit $R^1$ = $O-C(O)-CH_2-C(O)-CH_3$

oder daß man

l) eine Verbindung der Formel III mit einem Mesoxalsäurediester umsetzt, wobei eine Verbindung der Formel II entsteht mit

$A^1$ und $A^2$ = 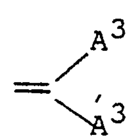

wobei $A^3$ und $A^{3'}$ wie in Anspruch 1 in Formel I und II definiert sind,

oder daß man

zwei oder mehrere der vorstehend genannten Verfahrensvarianten a-l hintereinander ausführt und daß man anschließend gegebenenfalls die Verbindungen I oder II in ihre physiologisch verträglichen Salze überführt.

**2.** Verfahren nach Anspruch 1, dadurch gekennzeichnet, daß man 10-Ring-Lactone der Formeln I und II in denen

$A^1$ und $A^2$

gleich oder verschieden sind und H oder $C_1$-$C_2$-Alkyl bedeuten, wobei der $C_1$-$C_2$-Alkylrest unsubstitu-

iert ist oder einfach substituiert ist mit Phenyl, wobei der Phenylrest seinerseits unsubstituiert ist oder einfach substituiert ist mit Halogen, $CF_3$ oder $C_1$-$C_3$-Alkyl
oder worin
$A^1$ und $A^2$
zusammen einen Rest der Formel

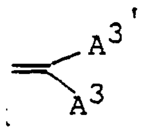

bilden, worin $A^3$ und $A^{3'}$ gleich oder verschieden sind und $C_1$-$C_2$-Alkoxycarbonyl oder Phenyl bedeuten
oder worin
$A^1$ und $A^2$
zusammen einen Rest der Formel

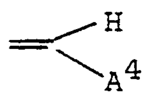

darstellen, worin $A^4$ Phenyl bedeutet,

$R^1$ -H, =O oder $-OR^3$ bedeutet und
$R^2$ =O, $-OR^4$, $=N-R^5$ oder 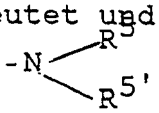

bedeutet, wobei im Falle der Formel II für $R^2$ = $=N-R^5$ und $A^1$ = H diese auch als Verbindung der Formel II', wie in Anspruch 1 angegeben, vorliegen kann
wobei
$R^3$
H, $C_1$-$C_8$-Alkyl, Benzyl, Allyl, MEM, MOM, SEM, THP, 4'-Methoxytetrahydropyran-4-yl, Trimethylsilyl, Triethylsilyl, Dimethylphenylsilyl, Dimethylcyclohexylsilyl, Dimethyltertiarbutylsilyl oder einen Sulfonsäureester der Formel $-SO_2R^7$ bedeutet, wobei $R^7$ Methyl, Phenyl oder p-Methylphenyl bedeutet
oder
$R^3$
einen Pyranosylrest darstellt, dessen OH-Gruppen ungeschützt sind oder mit Schutzgruppen geschützt sind
oder
$R^3$
einen Rest der Formel

$$-\overset{X}{\overset{\|}{C}}-Z-R^8 \quad \text{oder} \quad -\overset{X}{\overset{\|}{C}}-Z-(CH_2)_n-R^8$$

darstellt, wobei
X O

$$Z \quad O, \; -\overset{|}{N}-R^8, \; -\overset{|}{N}-(CH_2)_n-R^8 \; \text{oder} \; -\overset{\backslash}{N}H$$

n 1 bis 3 bedeutet und

$R^8$ $C_1$-$C_8$-Alkyl, $C_3$-$C_6$-Cycloalkyl oder Aryl bedeutet
oder
$R^3$
einen Rest der Formel

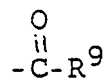

darstellt,
wobei
$R^9$
$C_1$-$C_{15}$-Alkyl, $C_2$-$C_{16}$-Alkenyl, $C_3$-$C_6$-Alkinyl, $C_3$-$C_6$-Cycloalkyl, Aryl oder Heteroaryl bedeutet, wobei die Alkyl-, Alkenyl-, Alkinyl- und Cycloalkylreste unsubstituiert sind oder ein- oder mehrfach substituiert sind mit $COOA^5$, Halogen, Phenyl Phenyloxy, $NHCOCH_3$, $COA^5$, $SO_3A^5$, wobei $A^5$ $C_1$-$C_4$-Alkyl oder H bedeutet
und wobei
alle genannten Aryl- und Heteroarylreste unsubstituiert sind oder ein- oder mehrfach substituiert sind mit Halogen, Cyano, Trifluormethyl, Carboxy, $C_1$-$C_4$-Alkyl-carbonyl, $C_1$-$C_4$-Alkoxy-carbonyl, $C_1$-$C_7$-Alkyl, $C_1$-$C_4$-Alkoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff, $C_1$-$C_{10}$-Alkyl, $C_2$-$C_{10}$-Alkenyl, Benzyl, Phenyl oder Phenylethyl bedeuten, wobei die genannten Reste unsubstituiert sind oder substituiert sind mit Hydroxy, Halogen, $NH_2$ oder $OCH_3$, sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H, herstellt

3. Verfahren nach Anspruch 1 oder 2, dadurch gekennzeichnet, daß man 10-Ring-Lactone der Formel I und II in denen
$A^1$ und $A^2$
gleich oder verschieden sind und H oder Methyl bedeuten, wobei der Methylrest unsubstituiert ist oder einfach substituiert ist mit Phenyl,
oder worin
$A^1$ und $A^2$
zusammen einen Rest der Formel

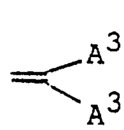

bilden, worin
$A^3$ und $A^{3'}$ gleich sind und
Äthoxycarbonyl bedeuten

$R^1$ -H, =O oder $-OR^3$ bedeutet und
$R^2$ =O, $-OR^4$, $=N-R^5$ oder 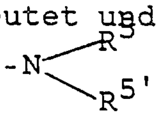

bedeutet, wobei im Falle der Formel II für $R^2$ = $=N-R^5$ und $A^1$ = H diese auch als Verbindung der

Formel II', wie in Anspruch 1 angegeben, vorliegen kann
wobei
$R^3$
H, Methyl, Benzyl, THP, Trimethylsilyl, Dimethyltertiärbutylsilyl oder einen Sulfonsäureester der Formel $-SO_2R^7$ bedeutet, wobei $R^7$ p-Methylphenyl bedeutet
oder
$R^3$
einen Glucosyl-, Galactosyl- oder Lactosylrest darstellt, dessen OH-Gruppen ungeschützt sind oder mit Acetylgruppen oder Benzylgruppen geschützt sind, wobei diese genannten Zuckerreste entweder glykosidisch gebunden sind oder über einen Orthoester verknüpft sind
oder
$R^3$
einen Rest der Formel

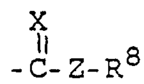 oder 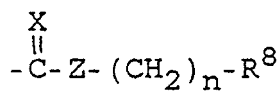

darstellt, wobei
X O,
Z -NH,
n 1 bedeutet und
$R^8$ $C_4$-$C_8$-Alkyl oder Aryl bedeutet
oder
$R^3$
einen Rest der Formel

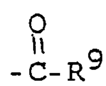

darstellt, wobei
$R^9$
$C_1$-$C_{15}$-Alkyl, $C_2$-$C_8$-Alkenyl, $C_3$-$C_6$-Cycloalkyl, Phenyl oder Thienyl oder Furyl bedeutet, wobei die Alkyl-, Alkenyl-, und Cycloalkylreste unsubstituiert sind oder ein- oder mehrfach substituiert sind mit $COOA^5$, F, Cl, Br,Phenyl, Phenyloxy, $NHCOCH_3$, $COA^5$, wobei $A^5$ Methyl oder H bedeutet und wobei alle genannten Aryl- oder Phenylreste unsubstituiert sind oder ein- oder mehrfach substituiert sind mit F, Cl, Br, Trifluormethyl, Carboxy, $C_1$-$C_4$-Alkyl-carbonyl, $C_1$-$C_4$-Alkoxy-carbonyl, $C_1$-$C_7$-Alkyl, Methoxy, $NHCOCH_3$
und worin
$R^4$
unabhängig von $R^3$ die oben für $R^3$ angegebenen Bedeutungen hat und
$R^5$ und $R^{5'}$
gleich oder verschieden sind und Wasserstoff, $C_1$-$C_6$-Alkyl, Benzyl, Phenyl oder Phenylethyl bedeuten, wobei die genannten Reste unsubstituiert sind oder substituiert sind mit F, Cl, Br oder $OCH_3$,
sowie die physiologisch verträglichen Salze,
ausgenommen die Verbindung der Formel I, in der $R^2$ $-OR^4$ bedeutet mit $R^4$ = H sowie ebenfalls ausgenommen die Verbindung der Formel II, in der $R^1$ $-OR^3$ und $R^2$ =O bedeutet mit $R^3$ = H, herstellt.

4. Verwendung der 10-Ring-Lactone der Formeln I und II nach einem oder mehreren der Ansprüche 1 bis 3, jedoch einschließlich der Verbindung der Formel I in der $R^2$ $-OR^4$ bedeutet mit $R^4$ = H, sowie einschließlich der Verbindung der Formel II, in der $R^1$ $-OR^3$ und $R^2$ =O bedeutet, mit $R^3$ = H, zur Herstellung eines Arzneimittels mit Cholesterin-Biosynthese hemmender Wirkung.

5. Verwendung der Verbindungen der Formel I und II nach einem oder mehreren der Ansprüche 1 bis 3

zur Herstellung eines Arzneimittels.

6. Verwendung der Verbindungen der Formeln I und II nach einem oder mehreren der Ansprüche 1 bis 3 zur Herstellung eines Arzneimittels mit lipidsenkender Wirkung.

7. Verfahren zur Herstellung einer pharmazeutischen Zubereitung, dadurch gekennzeichnet, daß man mindestens eine Verbindung der Formel I und II gemäß der Definition in einem oder mehreren der Ansprüche 1 bis 3, gegebenenfalls mit anderen Derivaten der Formel I, mit einem physiologisch annehmbaren Träger sowie gegebenenfalls geeigneten Zusatz- und/oder Hilfsstoffen in eine geeignete Darreichungsform bringt.

8. Verfahren zur Herstellung eines Arzneitmittels, dadurch gekennzeichnet, daß man in das Arzneimittel mindestens eine Verbindung der Formel I und/oder II nach einem der Ansprüche 1 bis 3 einverleibt.